# FASCICULE DE BREVET EUROPEEN

(11) **EP 2 989 144 B1**
(45) Date de publication et mention de la délivrance du brevet: **17.05.2017**
(21) Numéro de dépôt: 14722136.0
(22) Date de dépôt: 22.04.2014
(51) Int. Cl.: C08G 73/10, C08G 73/14, C08L 79/08, C07D 307/54

(54) **POLYIMIDES, PROCEDES DE FABRICATION DESDITS POLYIMIDES ET ARTICLES OBTENUS A PARTIR DESDITS POLYIMIDES**
POLYIMIDE, VERFAHREN ZUR HERSTELLUNG VON POLYIMIDEN UND DARAUS HERGESTELLTE GEGENSTÄNDE
POLYIMIDES, PROCESSES FOR PRODUCING SAID POLYIMIDES AND ARTICLES OBTAINED FROM SAID POLYIMIDES

(30) Priorité: 24.04.2013 FR 1353729
(43) Date de publication de la demande: 02.03.2016
(73) Titulaire: Rhodia Operations, 75009 Paris (FR)
(72) Inventeur: JEOL, Stéphane, Cumming, GA 30041 (US)
(74) Mandataire: Ridray, Annabelle
(86) Numéro de dépôt international: PCT/EP2014/058100
(87) Numéro de publication internationale: WO 2014/173876

(56) Documents cités:
- WO-A1-2013/007585
- LEILA BEN MAKTOUF ET AL: "Polyimides based on furanic diamines and aromatic dianhydrides: synthesis, characterization and properties", POLYMER BULLETIN, SPRINGER, BERLIN, DE, vol. 67, no. 7, 26 janvier 2011 (2011-01-26), pages 1111-1122, XP019957020, ISSN: 1436-2449, DOI: 10.1007/S00289-010-0441-4 cité dans la demande
- Stefan Löbbecke: "Reaktionsscreening im Mikroreaktor zur Herstellung von Synthesebausteinen aus nachwachsenden Rohstoffen", , 1 septembre 2009 (2009-09-01), XP055077587, Extrait de l'Internet: URL:http://www.dbu.de/OPAC/ab/DBU-Abschlus sbericht-AZ-23912.pdf [extrait le 2013-09-04]

## Description

La présente invention concerne de nouveaux (co)polymères et leurs procédés de synthèse. Plus précisément, la présente invention vise des (co)polymères comprenant au moins une fonction imide mettant en oeuvre une diamine particulière ou son dérivé diisocyanate. Ces (co)polymères peuvent être transformés en articles par diverses méthodes.

### ART ANTERIEUR

Les polyimides, notamment les polyimides aromatiques, sont connus pour leurs propriétés thermiques et/ou mécaniques exceptionnelles, ce qui les destine en particulier à des applications dites de « haute performance » dans différents domaines tels que l'aéronautique ou encore l'électronique (cartes de circuits imprimés par exemple). Ils sont généralement infusibles, c'est à dire qu'ils se dégradent avant de fondre (à plus de 500°C), et considérés comme des thermodurs, c'est-à-dire qu'une fois formés on ne peut pas les mettre en oeuvre par refusion. Ils se caractérisent généralement par des tenues mécaniques excellentes et des coefficients d'expansion thermique faible en raison des températures de transition vitreuse (Tg) très élevées, généralement supérieure à 200°C voire supérieure à 250°C.

Ces polyimides aromatiques sont généralement synthétisés selon un procédé en deux étapes : synthèse d'un polyacide amique à partir d'un dianhydride aromatique et d'une diamine aromatique, puis cyclisation en polyimide lors de la fabrication des articles. Cependant, ces polyimides aromatiques présentent un certain nombre d'inconvénients liés à l'emploi de diamines aromatiques, pour la plupart cancérigènes. En effet, la toxicité de ces diamines impose des conditions de manipulations très précautionneuses lors de la synthèse des polyacides amiques et également de veiller à contrôler la quantité de diamine aromatique libre dans les solutions de polyacide amique avant leur utilisation et sur le polyimide final.
Pour diminuer les risques liés à la manipulation de ces diamines, une alternative consiste à substituer partiellement ou totalement les diamines aromatiques (la fonction amine est liée directement par un atome de carbone faisant partie d'un cycle aromatique) par des diamines aliphatiques, cycloaliphatiques ou arylaliphatiques dont les fonctions amine sont liées à un atome de carbone ne faisant pas partie d'un cycle aromatique. Dans US7691471 des diamines cycloaliphatiques particulières permettant d'obtenir des polyimides de Tg élevées, par exemple supérieures à 250°C sont décrites. US3179614 décrit quant à lui des polyacides amiques précurseurs de polyimides obtenus par réaction entre l'anhydride pyromellitique et la méta-xylylène diamine (diamine arylaliphatique) ou encore entre l'anhydride pyromellitique et la 2,11-dodécanediamine (diamine aliphatique). Cependant les polyimides obtenus avec les diamines aliphatiques ont une température de transition vitreuse basse, inférieure à 200°C comme décrit dans US2710853, limitant ainsi les performances de ces polymères et donc leur potentiel dans les applications « haute performance ».

Par ailleurs, les polymères synthétisés à partir de monomères biosourcés présentent un intérêt important de nos jours car ils permettent de réduire l'empreinte environnementale. Il existe un grand nombre de combinaisons de monomères biosourcés ou de combinaisons de monomères biosourcés et issus de ressources fossiles qui peuvent être utilisées pour générer des polymères dits alors biosourcés. Certains de ces polymères biosourcés peuvent être utilisés pour remplacer des polymères issus de ressources fossiles.
Par « biosourcé », on entend qu'il s'agit d'une matière dérivée de ressources renouvelables. Une ressource renouvelable est une ressource naturelle, animale ou végétale, dont le stock peut se reconstituer sur une période courte à l'échelle humaine. Il faut en particulier que ce stock puisse se renouveler aussi vite qu'il est consommé.
A la différence des matériaux issus de matières fossiles, les matières premières renouvelables contiennent une proportion importante de ¹⁴C. Cette caractéristique peut notamment être déterminée par l'une des méthodes décrites dans la norme ASTM D6866, notamment selon la méthode par spectrométrie de masse ou la méthode par spectrométrie à scintillation liquide.
Ces ressources renouvelables sont généralement produites à partir de matière végétale cultivée ou non telle que les arbres, les plantes comme la canne à sucre, le maïs, le manioc, le blé, le colza, le tournesol, la palme, le ricin ou analogues, ou à partir de matière animale telle que les graisses (suif etc.).

Parmi les monomères biosourcés, un grand intérêt se manifeste autour des molécules portant un cycle furane, notamment les dérivés de l'acide 2,5-furanedicarboxylique, obtenu par exemple à partir d'hydroxyméthylfurfural (HMF) lui-même obtenu par exemple à partir des sucres, tel que le glucose.

Dans le domaine des polyimides biosourcés, l'article « Polyimides based on furanic diamines and aromatic dianhydrides : synthesis, characterization and properties », Polym. Bull. (2011) 67 :1111-1122, décrit des polyimides obtenus par polycondensation entre des dianhydrides aromatiques et des diamines difuraniques. Cependant, les polyimides obtenus sont à la fois amorphes et présentent une Tg très basse (143°C au maximum). Ces polymères ne pourraient donc être utilisés qu'à des températures inférieures à 150°C, ce qui écarte les applications de «haute performance » sur lesquelles se situent généralement les polyimides conventionnels et les applications en substitution de polymères amorphes de hautes température de transition vitreuse comme les polyetherimides connus sous le nom de Ultem® (Tg d'environ 220°C) commercialisés par Sabic ou encore le polyamideimide Torlon® de Solvay (Tg d'environ 275°C).

L'objectif de la présente invention est donc de trouver de nouveaux polymères qui répondent aux exigences des applications « haute performance », c'est-à-dire ayant de bonnes propriétés thermiques, mécaniques, diélectriques (isolantes), ainsi qu'une bonne stabilité dimensionnelle. De plus, les polymères devront présenter une Tg élevée. En outre, les polymères pourront être obtenus à partir d'une grande variété de monomères, présentant une toxicité faible, voire nulle, éco-amicaux, biosourcés, peu onéreux, largement disponibles et/ou faciles à synthétiser.

### INVENTION

Il vient d'être mis en évidence par la demanderesse des (co)polymères particuliers, qui satisfont en tout ou partie les objectifs précités.

La présente invention concerne ainsi un (co)polymère comprenant l'unité récurrente de formule I suivante : 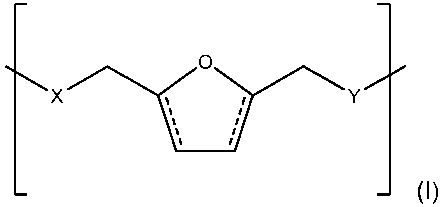 dans laquelle
- X est un groupe divalent de formule la ou Ib suivantes : 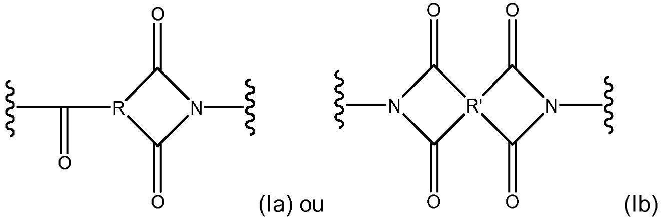 dans lesquelles
   - R est un groupe hydrocarboné trivalent choisi parmi:
      un groupe aliphatique ou cycloaliphatique, saturé ou insaturé, éventuellement interrompu par un ou plusieurs hétéroatomes, éventuellement substitué par un ou plusieurs groupements fonctionnels ou non ;
      un groupe aromatique, alkylaromatique ou arylaliphatique, éventuellement interrompu par un ou plusieurs hétéroatomes, éventuellement substitué par un ou plusieurs groupements fonctionnels ou non ;
   - R' est un groupe hydrocarboné tétravalent choisi parmi:
      un groupe aliphatique ou cycloaliphatique, saturé ou insaturé, éventuellement interrompu par un ou plusieurs hétéroatomes, éventuellement substitué par un ou plusieurs groupements fonctionnels ou non ;
      un groupe aromatique, alkylaromatique ou arylaliphatique, éventuellement interrompu par un ou plusieurs hétéroatomes, éventuellement substitué par un ou plusieurs groupements fonctionnels ou non ;
- Y est :
   - une liaison covalente, lorsque X répond à la formule Ib,
   - un groupe divalent de formule Ic ou Id suivantes, lorsque X répond à la formule Ia :
      ○ 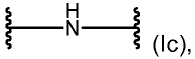 ou
      ○ 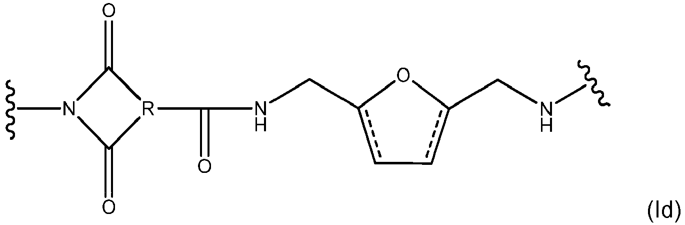 avec R tel que défini ci-dessus.

Le (co)polymère selon l'invention comprend donc au moins une fonction imide dans son unité récurrente de formule (I).

Ces (co)polymères peuvent être préparés en utilisant comme monomères constitutifs des diamines ou des diisocyanates spécifiques décrits ci-après.

Selon un premier mode de réalisation, le (co)polymère selon l'invention peut donc être obtenu par polymérisation des composés suivants :
- au moins un composé A comprenant deux fonctions anhydride et/ou ses équivalents acide tétracarboxylique et/ou ester; et
   - au moins une diamine B de formule II ou III, 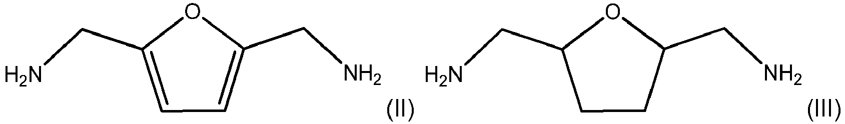 ou
   - au moins un diisocyanate B' de formule II' ou III' suivantes : 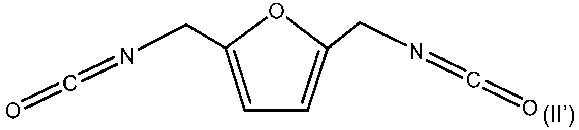 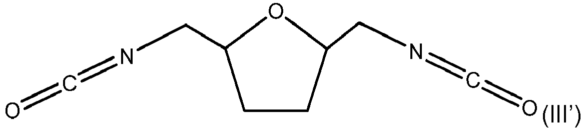
   ou
- au moins un sel de carboxylate d'ammonium obtenu à partir des composés A et B.

Selon un second mode de réalisation, le (co)polymère selon l'invention peut être obtenu par polymérisation des composés suivants :
- au moins un composé D comprenant une fonction anhydride et une fonction acide carboxylique et/ou ses équivalents acide tricarboxylique et/ou ester et/ou anhydride chlorure d'acide ; et
   - au moins une diamine B de formule II ou III, 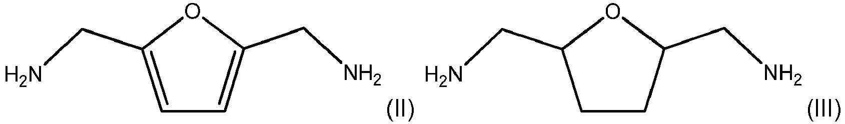 ou
   - au moins un diisocyanate B' de formule II' ou III' suivantes : 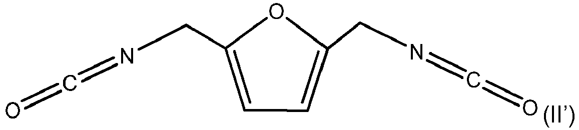 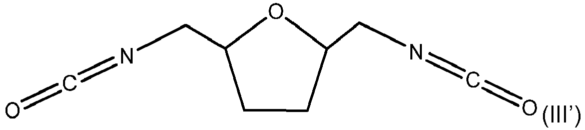
   ou
- au moins un sel de carboxylate d'ammonium obtenu à partir des composés D et B.

Le (co)polymère de l'invention peut aussi être obtenu par polymérisation entre au moins le composé A et au moins la diamine B ou au moins le diisocyanate B', en présence de :
- lorsque la diamine B est utilisée : au moins une diamine C de formule IV suivante :

   NH₂-R₃-NH₂ (IV)

   ou
- lorsque le diisocyanate B' est utilisé : au moins un diisocyanate C' de formule IV' suivante :

   O=C=N-R₃-N=C=O (IV')

   avec R₃ étant un radical divalent hydrocarboné aliphatique, cycloaliphatique ou arylaliphatique, saturé et/ou insaturé, aromatique ou alkylaromatique, qui comprend éventuellement des hétéroatomes.

Le (co)polymère de l'invention peut aussi être obtenu par polymérisation entre au moins le composé D et au moins la diamine B ou au moins le diisocyanate B', en présence de :
- lorsque la diamine B est utilisée : au moins une diamine C de formule IV suivante :

   NH₂-R₃-NH₂ (IV)

   ou
- lorsque le diisocyanate B' est utilisé : au moins un diisocyanate C' de formule IV' suivante :

   O=C=N-R₃-N=C=O (IV')

   avec R₃ étant un radical divalent hydrocarboné aliphatique, cycloaliphatique ou arylaliphatique, saturé et/ou insaturé, aromatique ou alkylaromatique, qui comprend éventuellement des hétéroatomes.

La présente invention concerne aussi un sel de carboxylate d'ammonium obtenu à partir d'un ou plusieurs composés A et une ou plusieurs diamines B et éventuellement une ou plusieurs diamines C, lesdits composés A, B et C étant tels que définies ci-dessus ou plus bas dans la description.

La présente invention concerne aussi un sel de carboxylate d'ammonium obtenu à partir d'un ou plusieurs composés D et une ou plusieurs diamines B et éventuellement une ou plusieurs diamines C, lesdits composés D, B et C étant tels que définies ci-dessus ou plus bas dans la description.

L'invention concerne aussi des procédés de préparation des (co)polymères selon l'invention par polymérisation entre au moins un composé A et au moins une diamine B ou au moins un diisocyanate B', éventuellement en présence d'au moins une diamine C lorsque la diamine B est utilisée ou d'au moins un diisocyanate C' lorsque le diisocyanate B' est utilisé, lesdits composés A, B, B', C et C' étant tels que définis ci-dessus ou plus bas dans la description.

L'invention concerne aussi des procédés de préparation des (co)polymères selon l'invention par polymérisation entre au moins un composé D et au moins une diamine B ou au moins un diisocyanate B', éventuellement en présence d'au moins une diamine C lorsque la diamine B est utilisée ou d'au moins un diisocyanate C' lorsque le diisocyanate B' est utilisé, lesdits composés D, B, B', C et C' étant tels que définis ci-dessus ou plus bas dans la description.

L'invention vise également des compositions comprenant le (co)polymère de l'invention et des charges et/ou additifs.

L'invention vise aussi des procédés de fabrication d'articles à base de (co)polymère selon l'invention, notamment :
- par mise en forme par extrusion, moulage ou soufflage dudit (co)polymère sous forme fondue lorsqu'il présente une température de fusion inférieure à 350°C pour un (co)polymère semi-cristallin ou une température de transition vitreuse inférieure à 300°C pour un (co)polymère amorphe, ou
- par cyclisation d'un intermédiaire polyacide amique tel que défini ci-dessous sous forme solide ou d'une solution ou d'une dispersion dans un solvant, le dit intermédiaire polyacide amique étant avantageusement obtenu par réaction entre les composés A et B, éventuellement en présence de diamine C.

L'invention vise également un polyacide amique, notamment obtenu par réaction entre les composés A et B, éventuellement en présence de diamine C, de formule V suivante : 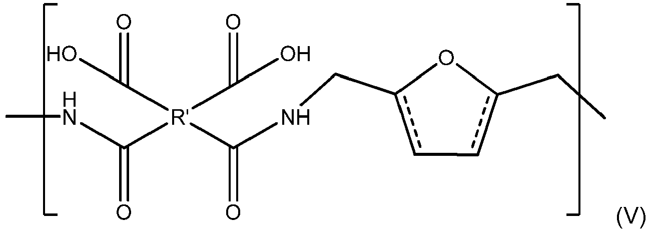 avec R' étant tel que défini précédemment.

Enfin, l'invention concerne donc un article obtenu à partir du (co)polymère selon l'invention ou de la composition le comprenant. Il peut s'agir d'une pièce moulée comme par exemple une pièce moulée par injection ou un composite à fibres continues, une pièce extrudée comme par exemple un film, une fibre, un fil, ou un filament, ou encore une pièce soufflée. Il peut également s'agir d'une mousse. Il peut aussi s'agir d'une pièce tissée ou tricotée à partir de fibres, fils ou filaments à base de (co)polymère selon l'invention.

### Définitions

On entend par « semi-cristallin », un (co)polymère présentant une phase amorphe et une phase cristalline, ayant par exemple un taux de cristallinité compris entre 1% et 85%. On entend par « amorphe » un (co)polymère ne présentant pas de phase cristalline détectée par les analyses thermiques (type DSC « Differential Scanning Calorimetry ») et en diffraction des rayons X.

On entend par « (co)polymère thermoplastique », un (co)polymère présentant une température au-delà de laquelle la matière se ramollit et fond sans se dégrader et qui, au dessous de celle-ci, devient dure.

Lorsque l'on prépare un (co)polymère à partir d'un sel de carboxylate d'ammonium, la détermination de la température de fusion du sel est préférentiellement effectuée par la mesure de la température de fin de l'endotherme mesurée par calorimétrie différentielle à balayage (DSC « Differential Scanning Calorimetry »), à l'aide d'un appareil Perkin Elmer Pyris 1, en chauffant le sel à partir de 20°C à une vitesse de 10°C/min.

La détermination de la température de fusion du (co)polymère est préférentiellement effectuée au pic de l'endotherme de fusion mesurée par calorimétrie différentielle à balayage (DSC « Differential Scanning Calorimetry »), à l'aide d'un appareil Perkin Elmer Pyris 1, en chauffant le (co)polymère à partir de 20°C à une vitesse de 10°C/min. La détermination de la température de transition vitreuse du (co)polymère est relevée au point moyen en utilisant la méthode des tangentes en chauffant le (co)polymère à partir de 20°C à une vitesse de 10°C/min.

Le (co)polymère selon l'invention comprend l'unité récurrente I, c'est-à-dire qu'il comprend au moins une fonction imide dans son unité récurrente I.

Dans le cas où le (co)polymère de l'invention comprend au moins une fonction imide et au moins une fonction amide dans son unité récurrente I (c'est-à-dire lorsque X = la et Y = Ic ou Id), on parle alors de (co)polyamideimide.

Dans le cas où le (co)polymère de l'invention comprend deux fonctions imides dans son unité récurrente 1 (c'est-à-dire lorsque X = Ib et Y est une liaison covalente), on parle de (co)polyimide.

Le (co)polymère selon l'invention peut être constitué essentiellement de l'unité récurrente de formule I. Il s'agit alors d'un homopolymère. Par essentiellement, on entend que l'unité récurrente de formule I représente au moins 95% du (co)polymère selon l'invention.
Le (co)polymère selon l'invention peut être constitué majoritairement de l'unité récurrente de formule I. Il s'agit alors d'un copolymère. Par majoritairement, on entend que l'unité récurrente de formule I représente au moins 50 % du (co)polymère selon l'invention.
Le (co)polymère selon l'invention peut comprendre minoritairement l'unité récurrente de formule I. Il s'agit alors d'un copolymère. Par minoritairement, on entend que l'unité récurrente de formule I représente moins de 50% du (co)polymère selon l'invention.

Les (co)polymères obtenus à partir d'une seule diamine B ou d'un seul diisocyanate B' et d'un seul composé A sont des homopolymères. Les (co)polymères obtenus à partir d'une seule diamine B ou d'un seul diisocyanate B' et d'un seul composé D sont des homopolymères.
La réaction entre au moins 3 monomères différents produit un copolymère. Les (co)polymères peuvent être définis par la composition molaire en chaque monomère constitutif.

### Les (co)polymères

Dans la formule I, les liaisons en pointillés signifient que le cycle est soit furanique (deux doubles liaisons) soit tétrahydrofuranique (deux simples liaisons). En d'autres termes, la formule I correspond à l'une des formules I.1 ou I.2 suivantes : 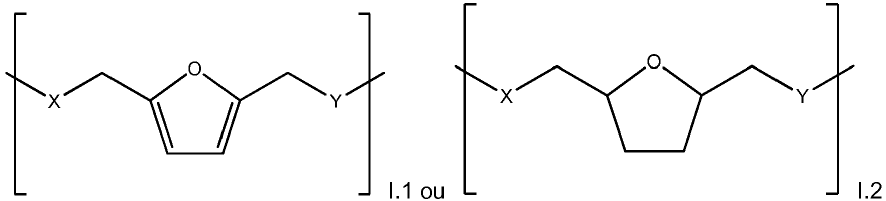 avec X et Y répondant aux définitions données ci-avant et plus bas dans la description.

Dans la formule V du polyacide amique selon l'invention, les liaisons en pointillés signifient également que le cycle est soit furanique (deux doubles liaisons) soit tétrahydrofuranique (deux simples liaisons).

Dans la formule I, R est un groupe hydrocarboné trivalent choisi parmi :
un groupe aliphatique ou cycloaliphatique, saturé ou insaturé, éventuellement interrompu par un ou plusieurs hétéroatomes, éventuellement substitué par un ou plusieurs groupements fonctionnels ou non ;
un groupe aromatique, alkylaromatique ou arylaliphatique, éventuellement interrompu par un ou plusieurs hétéroatomes, éventuellement substitué par un ou plusieurs groupements fonctionnels ou non.
Le groupe aliphatique ou cycloaliphatique, saturé ou insaturé, a avantageusement entre 1 et 18 atomes de carbone.
Le groupe aromatique, alkylaromatique ou arylaliphatique a avantageusement entre 4 et 18 atomes de carbone.
Par « éventuellement interrompu par un ou plusieurs hétéroatomes », on entend que le groupe hydrocarboné peut être interrompu par un ou plusieurs hétéroatomes suivants O, N, S ou P.
Par « substitué par un ou plusieurs groupements fonctionnels ou non », on entend que le groupe hydrocarboné peut être substitué par des groupes fonctionnels suivants : -OH, C=O, sulfonate, sulfonique, -COOH, halogène (-F, -CI, -Br) ; ou par des groupes non fonctionnels tels que des alkyles.

Dans la formule I, R' est un groupe hydrocarboné tétravalent choisi parmi :
un groupe aliphatique ou cycloaliphatique, saturé ou insaturé, éventuellement interrompu par un ou plusieurs hétéroatomes, éventuellement substitué par un ou plusieurs groupements fonctionnels ou non ;
un groupe aromatique, alkylaromatique ou arylaliphatique, éventuellement interrompu par un ou plusieurs hétéroatomes, éventuellement substitué par un ou plusieurs groupements fonctionnels ou non.
Le groupe aliphatique ou cycloaliphatique, saturé ou insaturé, a avantageusement entre 1 et 18 atomes de carbone.
Le groupe aromatique, alkylaromatique ou arylaliphatique a avantageusement entre 4 et 18 atomes de carbone.
Par « éventuellement interrompu par un ou plusieurs hétéroatomes », on entend que le groupe hydrocarboné peut être interrompu par un ou plusieurs hétéroatomes suivants O, N, S ou P.
Par « substitué par un ou plusieurs groupements fonctionnels ou non », on entend que le groupe hydrocarboné peut être substitué par des groupes fonctionnels suivants : -OH, C=O, sulfonate, sulfonique, -COOH, halogène (-F, -CI, -Br) ; ou par des groupes non fonctionnels tels que des alkyles.

Les (co)polymères de l'invention présentent avantageusement une température de transition vitreuse Tg supérieure à 150°C, en particulier supérieure à 200°C.
Lorsqu'un polymère présente une Tg supérieure à 150°C, cela signifie que lorsque les articles faits avec ce polymère sont utilisés à une température inférieure à 150°C, le polymère est dans son état vitreux, c'est-à-dire l'état dans lequel il est le plus rigide. Il y a un grand nombre d'applications dans lesquelles la température d'utilisation de ces articles n'excède pas 150°C et plus particulièrement 200°C : par exemple un habitacle dans l'automobile, les bâtiments, l'emballage, l'électrique, l'électronique, ... Le dimensionnement d'une pièce est effectué à la température à laquelle l'article va être utilisé. Ainsi, si on utilise un polymère semi-cristallin dont la Tg est supérieure à la température d'utilisation, les calculs prennent en compte la rigidité élevée du polymère. Par rapport à un polymère semi-cristallin dont la Tg serait inférieure à la température d'utilisation, on peut donc se permettre le cas échéant d'utiliser moins de matière.

Tout particulièrement, les (co)polymères de l'invention présentent une Tg inférieure ou égale à 300°C. Ceci peut notamment permettre une mise en oeuvre plus facile.

Les (co)polymères selon l'invention peuvent être semi-cristallins ou amorphes. S'ils sont semi-cristallins, les (co)polymères selon l'invention peuvent ainsi présenter une température de fusion Tf allant de 100 à 350°C, en particulier de 150°C à 350°C. Généralement, lorsque le (co)polymère selon l'invention est un (co)polyamideimide, c'est-à-dire qu'il comprend au moins une fonction imide et au moins une fonction amide dans son unité récurrente I (lorsque X = la et Y = Ic ou Id), alors il s'agit d'un polymère thermoplastique, qui peut donc être transformé en articles par voie fondu selon les procédés classiquement utilisés pour les polymères thermoplastiques tels que le PA66. Lorsque le (co)polymère selon l'invention est un (co)polyimide, c'est-à-dire qu'il comprend deux fonctions imides dans son unité récurrente I (lorsque X = Ib et Y est une liaison covalente), alors il est généralement non transformable par voie fondue.

Les (co)polymères amorphes présentent l'avantage d'être transparents (lorsqu'il ne sont pas formulés), ce qui est important en optique. Pour pouvoir être utilisés, ces polymères doivent impérativement présenter une Tg supérieure à la température d'utilisation.
Les (co)polymères semi-cristallins présentent l'avantage de conserver des propriétés mécaniques au-delà de leur Tg, jusqu'à leur Tf ou leur température de dégradation.

Les (co)polymères selon l'invention présentent avantageusement un masse molaire moyenne en nombre Mn allant de 1000 à 100000 g/mol, préférentiellement de 2000 à 50000 g/mol et encore plus préférentiellement de 5000 à 30000 g/mol.

Les (co)polymères de l'invention peuvent présenter une excellente stabilité dimensionnelle, c'est-à-dire qu'ils présentent un coefficient linéaire thermique d'expansion CLTE faible, notamment se rapprochant de celui du Kapton ®.

### Monomères

### Les composés A

L'un des monomères constitutifs du (co)polymère selon l'invention, peut être au moins un composé A comprenant deux fonctions anhydride et/ou ses équivalents acide tétracarboxylique et/ou ester.

Par « équivalents » ou « dérivés », on comprendra au sens de la présente invention, que les « équivalents ou dérivés acides tétracarboxyliques » sont les dianhydrides hydrolysés. Pour les « équivalents ou dérivés esters », on comprendra au sens de la présente invention, qu'il s'agit des esters des équivalents/dérivés acides tétracarboxyliques, une ou plusieurs fonctions acide étant estérifiée par un alcool.

Les composés A portent préférentiellement des fonctions acides carboxyliques dans des positions telles qu'elles permettent de former deux fonctions anhydrides d'acides sur une même molécule (par une réaction de déshydratation). Les composés A présentent généralement deux paires de fonctions acides carboxyliques, chaque paire de fonction étant liée à un atome carbone adjacent, en α et β.
Les quatre fonctions acides carboxyliques équivalentes peuvent être obtenues à partir de dianhydrides d'acides par hydrolyse des fonctions anhydrides. Des exemples de dianhydrides d'acides et d'acides tétracarboxyliques, dérivés des dianhydrides, sont décrits dans le brevet US7932012.

Les composés A de l'invention peuvent également porter au moins un autre groupe fonctionnel. Ce groupe peut notamment être choisi parmi le groupe -SO₃X, avec X=H ou un cation, notamment Na, Li, Zn, Ag, Ca, Al, K et Mg, le groupe hydroxyle -OH, le groupe cétone C=O, et les éthers -O-.

Les composés A comprenant deux fonctions anhydride sont préférentiellement choisis dans le groupe constitué par: le dianhydride éthane-1,1,2,2-tétracarboxylique, le dianhydride butane-1,2,3,4-tétracarboxylique, le dianhydride pentane-1,2,4,5-tétracarboxylique, le dianhydride cyclobutane-1,2,3,4-tétracarboxylique, le dianhydride cyclopentane-1,2,3,4-tétracarboxylique, le dianhydride cyclohexane-1,2,4,5 tétracarboxylique, le dianhydride cyclohexane-2,3,5,6-tétracarboxylique, le dianhydride 3-éthyl-cyclohexane-3-(1,2)5,6- tétracarboxylique, le dianhydride 1-méthyl-3-éthyl cyclohexane-3-(1,2)5,6-tétracarboxylique, le dianhydride 1-éthyl-cyclohexane-1-(1,2),3,4-tétracarboxylique, le dianhydride 1-propyl-cyclohexane-1-(2,3),3,4-tétracarboxylique, le dianhydride 1,3-dipropyl-cyclohexane-1-(2,3),3-(2,3)-tétracarboxylique, le dianhydride dicyclohexyl-3,4,3',4'-tétracarboxylique, le dianhydride tétrahydrofurane-2,3,4,5-tétracarboxylique, l'anhydride pyromellitique, le dianhydride 3,3',4,4'-biphényltétracarboxylique, le dianhydride 2,3,3',4'-biphényltétracarboxylique, le dianhydride 2,2',3,3'-biphényl-tétracarboxylique, le dianhydride 3,3',4,4'-benzophénone-tétracarboxylique, le dianhydride 2,2',3,3'-benzophénone-tétracarboxylique, le dianhydride 1,2,5,6-naphthalènetétracarboxylique, le dianhydride 2,3,6,7-naphtalène-tétracarboxylique, le dianhydride 2,2'-bis-(3,4-dicarboxyphényl) hexafluoropropane tétracarboxylique, le dianhydride 4,4'-oxydiphtalique, le dianhydride 2,2-bis(3,4-dicarboxyphénol) sulfone, le dianhydride de Bisphénol A, l'anhydride 4,4'-(hexafluoroisopropylidène)-diphthalique (6FDA), le dianhydride 3,4,9,10-pérylènetétracarboxylique, le dianhydride 3,3',4,4'-diphénylsulfone tétracarboxylique, le dianhydride 2,3,6,7-naphtalène tétracarboxylique, l'éthylèneglycol bis(trimellitic anhydride), l'anhydride hydroquinonediphthalique, le dianhydride 2,2-bis(3,4-dicarboxyphényl)propane, le dianhydride 1,1-bis(2,3-dicarboxyphényl)éthane, le dianhydride 1,1-bis(3,4-dicarboxyphényl)éthane, le dianhydride bis(2,3-dicarboxyphényl) méthane, le dianhydride bis(3,4-dicarboxyphényl)méthane, le dianhydride bis(3,4-dicarboxyphényl)sulfoxyde, le dianhydride pyrazine-2,3,5,6-tétracarboxylique, le dianhydride thiophène-2,3,4,5-tétracarboxylique, le dianhydride phénanthrène-1,8,9,10-tétracarboxylique et leurs mélanges.

Les composés A comprenant deux fonctions anhydride préférés sont choisis parmi l'anhydride pyromellitique, le dianhydride 3,3',4,4'-biphényl-tétracarboxylique, le dianhydride 4,4'-oxydiphtalique, le dianhydride 3,3',4,4'-benzophénone-tétracarboxylique, le dianhydride 2,2',3,3'-benzophénone-tétracarboxylique, le dianhydride éthane-1,1,2,2-tétracarboxylique, le dianhydride butane-1,2,3,4-tétracarboxylique, et leurs mélanges. Ces composés, en combinaison avec les diamines B ou les diisocyanates B' de l'invention, peuvent présenter l'avantage de donner des (co)polymères dont la Tg est supérieure à 150°C.

Parmi les dianhydrides cités ci-dessus, l'anhydride pyromellitique est particulièrement intéressant, notamment car il est peu onéreux, il permet d'obtenir des polyimides de Tg très élevée, il est largement disponible et il est facile à synthétiser.

Lorsque le composé A est un acide tétracarboxylique, notamment dérivé d'un composé comprenant deux fonctions anhydride, il est préférentiellement choisi dans le groupe constitué par: l'acide 1,2,3,4-butane tétracarboxylique, l'acide éthane-1,1,2,2-tétracarboxylique, l'acide pentane-1,2,4,5-tétracarboxylique, l'acide 1,2,3,4-cyclobutane-tétracarboxylique, l'acide cyclopentane-1,2,3,4-tétracarboxylique; l'acide cyclohexane-1,2,4,5-tétracarboxylique, l'acide cyclohexane-2,3,5,6-tétracarboxylique, l'acide 3-éthyl-cyclohexane-3-(1,2)5,6-tétracarboxylique, l'acide 1-méthyl-3-éthyl-cyclohexane-3-(1,2)5,6-tétracarboxylique, l'acide 1-éthyl-cyclohexane-1-(1,2),3,4-tétracarboxylique, l'acide 1-propyl-cyclohexane-1-(2,3),3,4-tétracarboxylique, l'acide 1,3-dipropylcyclohexane-1-(2,3),3-(2,3)-tétracarboxylique, l'acide dicyclohexyl-3,4,3',4'-tétracarboxylique, l'acide tétrahydrofuran-2,3,4,5-tétracarboxylique, l'acide pyromellitique, l'acide 3,3',4,4'-biphényltétracarboxylique, l'acide 2,3,3',4'-biphényltétracarboxylique, l'acide 2,2',3,3'-biphényltétracarboxylique, l'acide 3,3',4,4'-benzophénonetétracarboxylique, l'acide 2,2',3,3'-benzophénone-tétracarboxylique, l'acide 1,2,5,6-naphtalène-tétracarboxylique, l'acide 2,3,6,7-naphtalène-tétracarboxylique, l'acide 2,3,5,6-pyridine-tétracarboxylique, l'acide 3,4,9,10-pérylènetétracarboxylique, l'acide 3,3',4,4'-tétraphénylsilane-tétracarboxylique, l'acide 2,2'-bis-(3,4-bicarboxyphényl) hexafluoropropane tétracarboxylique, l'acide 4,4'-oxydiphtalique, l'acide 2,2-bis(3,4-dicarboxyphénol) sulfone, l'acide 4,4'-(hexafluoroisopropylidène)diphthalique, l'acide 3,4,9,10-pérylènetétracarboxylique, l'acide 3,3',4,4'-diphénylsulfone tétracarboxylique, l'acide 2,3,6,7-naphtalène tétracarboxylique, l'acide éthylèneglycol bistrimellitique, l'acide hydroquinonediphthalique, le 2,2-bis(3,4-dicarboxyphényl) propane, le 1,1-bis(2,3-dicarboxyphényl) éthane, le 1,1-bis(3,4-dicarboxyphényl) éthane, le bis(2,3-dicarboxyphényl) méthane, le bis(3,4-dicarboxyphényl) méthane, le bis(3,4-dicarboxyphényl) sulfoxyde, l'acide pyrazine-2,3,5,6-tétracarboxylique, l'acide thiophène-2,3,4,5-tétracarboxylique, l'acide phénanthrène-1,8,9,10-tétracarboxylique et leurs mélanges.

Avantageusement, lorsque le composé A est un acide tétracarboxylique, notamment dérivé d'un composé comprenant deux fonctions anhydride, il est préférentiellement choisi dans le groupe constitué par : l'acide pyromellitique, l'acide 3,3',4,4'-biphényltétracarboxylique, l'acide 4,4'-oxydiphtalique, l'acide 3,3',4,4'-benzophénonetétracarboxylique, l'acide 2,2',3,3'-benzophénone-tétracarboxylique, l'acide éthane-1,1,2,2-tétracarboxylique, l'acide butane-1,2,3,4-tétracarboxylique et leurs mélanges.

Parmi les tétraacides (ou acides tétracarboxyliques) cités ci-dessus, l'acide pyromellitique est particulièrement intéressant.

Alternativement, les composés A de l'invention peuvent être les esters des dianhydrides ou des tétraacides obtenus par réaction du dianhydride ou du tétraacide avec un monoalcool tel que le méthanol, l'éthanol, le propanol et isomères, le butanol et leurs isomères. Il peut s'agir de monoesters triacides, de diesters (ou hemiesters) diacides, de triesters monoacides ou de tétraesters. On préfère les diesters diacides, et en particulier le diester de l'acide pyromellitique obtenus par alcoolyse de l'anhydride pyromellitique par deux molécules d'alcool.

Dans un mode particulièrement préféré de réalisation de l'invention, les composés A sont des dianhydrides ou des acides tétracarboxyliques puisque ces composés présentent l'avantage de ne pas dégager de produits secondaires de réaction autres que de l'eau, et en particulier ils ne dégagent pas de solvants tels que des alcools. En particulier, on préfère l'anhydride pyromellitique ou l'acide pyromellitique.
De façon avantageuse, ces composés A préférés (l'anhydride pyromellitique ou l'acide pyromellitique) représentent au moins 80% molaire par rapport à l'ensemble des composés A mis en oeuvre.

Certains composés A peuvent présenter l'avantage d'être biosourcés, comme par exemple l'acide butane-1,2,3,4-tétracarboxylique.

### Les composés D

Les composés D sont choisis avantageusement parmi :
- les anhydrides monoacide tel que l'anhydride trimellitique,
- les triacides carboxyliques tels que l'acide trimellitique, l'acide tricarballylique, l'acide citrique, l'acide aconitique, l'acide 1,2,4-butane tricarboxylique, l'acide 1,2,3-benzène tricarboxylique,
- les monoester diacides, les diesters monoacide ou triesters des triacides carboxyliques ci-dessus,
- les anhydrides chlorure d'acide tel que le chlorure d'anhydride trimellitique.

### Les diamines B ou les diisocyanate B'

Selon l'invention, le terme « ou » utilisé entre les termes « diamine(s) B » et « diisocyanate(s) B' » signifie que les diamines B ne sont pas utilisées en combinaison avec les diisocyanates B'. Les diisocyanates B' sont une alternative aux diamines B.

Les diamines B selon l'invention sont choisies parmi le 2,5-bis(aminométhyl)furane et le 2,5-bis(aminométhyl)tétrahydrofurane.
Les diisocyanates B' selon l'invention sont choisis parmi les composés de formules II' et III'.
Dans le mode particulier de réalisation selon lequel la diamine B est le 2,5-bis-(aminométhyl)tétrahydrofurane ou le diisocyanate B' est la molécule de formule III', il peut s'agir du stéréoisomère cis ou trans ou d'un mélange de ceux-ci. Pour le stéréoisomère cis, les carbones chiraux en position 2 et 5 peuvent être R,S ou S,R ou un mélange méso. Pour le stéréoisomère trans, les carbones chiraux en position 2 et 5 peuvent être S,S ou R,R ou le mélange racémique.
Ces diamines B, avantageusement biosourcées, peuvent être synthétisées par exemple pour le 2,5-bis(aminométhyl)furane par nitrilation de l'acide 2,5-furanedicarboxylique suivie d'une hydrogénation sélective, et d'une hydrogénation du cycle furanique du 2,5-bis(aminométhyl)furane pour préparer le 2,5-bis(aminométhyl)tétrahydrofurane.
Ces diisocyanates B', avantageusement biosourcés, peuvent être synthétisés par exemple pour la molécule de formule III', par réaction du 2,5-bis(aminométhyl)tétrahydrofurane avec du phosgène selon les procédés connus pour transformer les diamines en diisocyanate.

### Les diamines C ou les diisocyanate C'

Selon l'invention, le terme « ou » utilisé entre les termes « diamine(s) C » et « diisocyanate(s) C' » signifie que les diamines C ne sont pas utilisées en combinaison avec les diisocyanates C'. Les diisocyanates C' sont une alternative aux diamines C.
De même, selon l'invention, les diisocyanates C' ne sont pas utilisés lorsque l'on utilise la diamine B, et inversement, les diamines C ne sont pas utilisés lorsque l'on utilise les diisocyanates B'.

Dans les formules IV ou IV' suivantes :

NH₂-R₃-NH₂ (IV)

O=C=N-R₃-N=C=O (IV')

R₃ est un radical divalent hydrocarboné aliphatique, cycloaliphatique ou arylaliphatique, saturé et/ou insaturé, aromatique ou alkylaromatique, qui comprend éventuellement des hétéroatomes.

Le radical R₃ comprend généralement de 2 à 100 atomes de carbone, préférentiellement de 4 à 50 atomes de carbone. Le radical R₃ peut éventuellement contenir un ou plusieurs hétéroatomes, tel que O, N, P ou S. le radical R₃ peut comprendre un ou plusieurs groupes fonctionnels comme des fonctions hydroxyles, sulfones, cétones, éthers, amines secondaires, amines tertiaires ou autres.

Les diamines C ou les diisocyanates C' peuvent notamment être des diamines/diisocyanates en positions alpha, oméga contenant de 4 à 20 groupes méthylènes.

Les diamines C aliphatiques peuvent par exemple être choisies dans le groupe comprenant: le 1,2-diaminoéthane, le 1,3-diaminopropane, le 1,4-diaminobutane, le 1,5-diaminopentane, l'hexaméthylène diamine, la 2,2,4- et 2,4,4-triméthyl-hexaméthylène diamine, le 1,7-diaminoheptane, le 1,8-diaminooctane, la 2-méthyl-1,8-diaminooctane, la 2,2,7,7-tétraméthyl-octaméthylène diamine, le 1,9-diaminonane, la 5-méthyl-1,9-diaminononane, le 1,10-diaminodécane, le 1,11-diaminoundécane, le 1,12-diaminododécane, le 1,13-diaminotridécane, le 1,14-diaminotétradécane, les diamines issues de dimères d'acide gras en C36 connus par exemple sous le nom PRIAMINE™ (référence 1075) commercialisée par CRODA.

Les diamines C cycloaliphatiques sont par exemple choisies dans le groupe comprenant l'isophorone diamine, le 1,3-bis(aminomethyle)cyclohexane, le 1,4-bis(aminomethyle)cyclohexane, le 1,3-diaminocyclohexane, le 1,4-diaminocyclohexane, et le diaminodicyclohexyl-méthane.
Les diamines arylaliphatiques sont par exemple la méta-xylylène diamine, para-xylylène diamine.

On peut citer des exemples de diamines C contenant des hétéroatomes comme les polyétherdiamines telles que les Jeffamine® et Elastamine® commercialisées par Hunstman. Il existe une variété de polyéther, composés de motifs oxyde d'éthylène, oxyde de propylène, oxyde de tétraméthylène. La masse molaire moyenne en nombre Mn est comprise entre 100 et 5000 g/mol.

Les diamines C aromatiques sont par exemple choisies dans le groupe suivant : diaminodiphénylméthane et ses isomères, la sulfonyldianiline et ses isomères, 3,4'-oxydianiline; 1,3-bis-(4-aminophénoxy)benzène; 1,3-bis-(3-aminophenoxy)benzène ; 4,4'-oxydianiline; 1,4-diaminobenzène; 1,3-diaminobenzène; 1 ,2-diaminobenzene, 2,2'-bis(trifluorométhyl)benzidène; 4,4'-diaminobiphényl; 4,4'-diaminodiphényl sulfide; 9,9'-bis(4-amino)fluorène; 4,4'-diaminodiphényl propane; 4,4'-diaminodiphényl méthane; benzidine; 3,3'-dichlorobenzidine; 3,3'-diaminodiphényl sulfone; 4,4'-diaminodiphényl sulfone; 1,5-diamino naphtalène; 4,4'-diaminodiphényl diéthylsilane; 4,4'-diamino diphénysilane; 4,4'-diaminodiphényl éthyl phosphine oxide; 4,4'-diamino diphényl N-méthyl amine; 4,4'-diamino diphényl N-phényl amine.

Avantageusement, la diamine C est choisie parmi : la méta-phénylène diamine, la para-phénylène diamine, l'hexaméthylènediamine, 2-méthylpentamethylène-diamine, le 1,10-diaminodécane, le 1,12-diaminododécane, la méta-xylylènediamine, le diaminodiphénylméthane et ses isomères, la sulfonyldianiline et ses isomères.

Les diisocyanates C' sont avantageusement choisis parmi : l'isophorone diisocyanate (IPDI), l'hexaméthylène diisocyanate (HDI), le toluène diisocyanate (TDI) et le méthylène diphényl diisocyanate (MDI), préférentiellement les isomères 2,2', 2,4' et 4,4'

Avantageusement, lorsque l'on utilise un mélange de diamines B et C, la diamine B représente au moins 60% molaire par rapport à l'ensemble des diamines B et C mises en oeuvre, de préférence au moins 80% molaire.

Avantageusement, lorsque l'on utilise un mélange de diisocyanates B' et C', le diisocyanate B' représente au moins 60% molaire par rapport à l'ensemble des diisocyanates B' et C' mis en oeuvre, de préférence au moins 80% molaire.

### Modes particuliers avec les diamines B et C

Selon un mode particulier de réalisation de l'invention, le (co)polymère selon l'invention est obtenu par polymérisation d'anhydride pyromellitique et du 2,5-bis(aminométhyl)tétrahydrofurane. Le polyimide ainsi obtenu est particulièrement avantageux puisqu'il présente une Tg très élevée, capable de rivaliser avec des polyimides non biosourcés de structure cycloaliphatique.

Selon un deuxième mode de réalisation de l'invention, le (co)polymère selon l'invention est obtenu par polymérisation d'anhydride trimellitique et du 2,5-bis(aminométhyl)tétrahydrofurane. Le polyamideimide ainsi obtenu est particulièrement avantageux puisqu'il présente une Tg très élevée et est thermoplastique.

Selon un troisième mode de réalisation de l'invention, le (co)polymère selon l'invention est obtenu par polymérisation d'acide pyromellitique, de 2,5-bis(aminométhyl)tétrahydrofurane et d'hexaméthylène 1,6-diamine.

Selon un quatrième mode de réalisation de l'invention, le (co)polymère selon l'invention est obtenu par polymérisation d'acide pyromellitique, de 2,5-bis(aminométhyl)tétrahydrofurane et de 1,10-décanediamine.

Selon un autre mode de réalisation de l'invention, le (co)polymère selon l'invention est obtenu par polymérisation de 3,3',4,4'-benzophénonetétracarboxylique dianhydride et de 2,5-bis(aminométhyl)tétrahydrofurane.

### Modes particuliers avec les diisocyanates B' et C'

Selon un mode particulier de réalisation de l'invention, le (co)polymère selon l'invention est obtenu par polymérisation d'anhydride trimellitique, du composé de formule III' et de l'isophorone diisocyanate.

Selon un autre mode particulier de réalisation de l'invention, le (co)polymère selon l'invention est obtenu par polymérisation d'anhydride trimellitique, du composé de formule III' et de MDI.

Selon un autre mode particulier de réalisation de l'invention, le (co)polymère selon l'invention est obtenu par polymérisation d'anhydride trimellitique, du composé de formule III' et de TDI.

### Les sels

La présente invention concerne aussi un sel de carboxylate d'ammonium obtenu à partir d'un ou plusieurs composés A (ou D) et une ou plusieurs diamines B et éventuellement une ou plusieurs diamines C, lesdits composés A (ou D), B et C étant tels que définis ci-dessus.

Le sel selon l'invention peut comprendre en outre au moins un composé limiteur de chaîne choisi parmi les monoamines, les monoacides ou les diacides en positions α,β tels qu'ils peuvent former une fonction anhydride par réaction de déshydratation. Avantageusement, le composé limiteur de chaîne est choisi parmi : le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges.
On préfère le 1-aminohexane, le 1-aminododécane, la benzylamine ou leurs mélanges. Ces composés ont pour effet de limiter la masse molaire du (co)polymère et ainsi de limiter la viscosité fondu du c(o)polymère, ce qui le rend plus aisément transformable par refusion pour réaliser des articles.

La teneur en limiteur de chaîne peut aller de 0,1 à 10 % en nombre de moles, notamment de 1 à 5% en nombre de moles, par rapport au nombre total de moles de monomères, c'est à dire acide tétracarboxylique, diamine et limiteur de chaine.

### Les procédés de synthèse

Plusieurs procédés de fabrication des (co)polymères selon l'invention par polymérisation des monomères A (ou D) et B, et éventuellement C ou A (ou D) et B', et éventuellement C' sont possibles, permettant d'obtenir le (co)polymère sous différentes formes (poudre thermoplastique ou non, pièce obtenue à partir de la cyclisation d'un précurseur du type polyacide amique, ...).

Il est par exemple possible de réaliser une polymérisation en solution (ou voie solvant), notamment en suivant les voies traditionnelles de synthèse des polyimides en solvant, par exemple en 2 étapes en passant par l'intermédiaire d'un polyacide amique (PAM). On peut également effectuer une polymérisation à l'état fondu ou à l'état solide, de mélanges de monomères ou à partir de sels de ces monomères.

De préférence, on choisira la synthèse à partir des polyacides amiques, qui permet la fabrication de pièces en polyimide à partir du chauffage de solution des polyacide amique à une température inférieure à la température de dégradation du polyimide.

### Synthèse en solution (passage par PAM), à partir des diamines et des dianhydrides (composés A dianhydrides)

Le procédé de synthèse des (co)polymères en voie solvant est un procédé qui comprend les étapes suivantes :
- on fait réagir à basse température, entre -20 et 120°C, préférentiellement entre 20 et 80°C, dans un solvant aprotique polaire ou un solvant phénolique au moins un composé A comprenant deux fonctions anhydride avec une diamine B, et éventuellement en présence d'au moins une diamine C, lesdits composés A, B et C étant tels que définis précédemment, pour former un intermédiaire appelé polyacide amique. Le temps de réaction peut être compris entre 5 min et 100 heures, préférentiellement entre 10 min et 10 heures ;
- on chauffe à une température comprise entre 120°C et 350°C, préférentiellement entre 140°C et 300°C, et/ou on effectue une déshydratation chimique en utilisant par exemple de l'anhydride acétique ;
- on récupère le (co)polymère, en fonction de sa solubilité dans le solvant, soit par évaporation du solvant dans le cas où le polymère est soluble dans le solvant, soit par précipitation dans un non solvant.
De préférence, le solvant aprotique polaire est choisi parmi le diméthyle acétamide, le diméthyle formamide, les crésols ou encore la N-méthylpyrrolidone.
De préférence, le solvant phénolique est choisi parmi le phénol, le 4-méthoxyphénol, le 2,6-diméthylphénol et le m-crésol.

Lors de la première étape, les amines ouvrent les cycles anhydride et donnent lieu à une fonction amide acide appelée souvent acide amique. Le polyacide amique formé est généralement soluble dans le solvant de synthèse et est transformé par cyclisation en (co)polymère qui le plus souvent est insoluble.

Le contrôle de la masse molaire moyenne en nombre peut être obtenu :
- par l'utilisation de limiteurs de chaînes, c'est-à-dire des molécules choisies parmi les monoamines, les monoanhydrides, les monoacides ou les diacides en positions α,β tels qu'ils peuvent former une fonction anhydride par réaction de déshydratation, parmi les limiteurs de chaîne on peut citer l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,
- par un déséquilibre stoechiométrique r=[composés A]/[diamines B + C],
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à tout moment du procédé de fabrication.

En particulier le déséquilibre stoechiométrique r peut aller de 0,8 à 1,2.

La teneur en limiteur de chaîne peut aller de 0,1 à 10 % en nombre de moles, notamment de 1 à 5% en nombre de moles, par rapport au nombre total de moles de monomères, c'est à dire dianhydride tétracarboxylique, diamine et limiteur de chaine.

Dans ce procédé, des catalyseurs, des charges inorganiques inertes ou réactives (argiles, silices ou précurseurs de silice, nanoparticules, ...), des stabilisants, des mâtifiants ou des colorants peuvent également être introduits.

Par exemple, pour faire un film en (co)polymère, il est possible de verser une solution de polyacide amique sur une surface chauffante. Lors du chauffage de la surface chauffante, le solvant s'évapore et la cyclisation se fait permettant l'obtention d'un film de (co)polymère.

Le (co)polymère peut être soluble ou non dans le solvant. Si le (co)polymère n'est pas soluble dans le solvant, le (co)polymère peut être obtenu par chauffage de la solution de polyacide amique et précipiter. Il peut ainsi être récupéré par filtration et séchage : une poudre est obtenue. Si le (co)polymère est soluble dans le solvant, il peut être récupéré sous la forme de poudre par précipitation dans ou avec un non solvant.
Des exemples de polymérisations selon la voie polyacide amique sont décrits dans le brevet US 2009/0263640.

### Synthèse en solution, à partir des diisocyanate et des dianhydrides A ou des diisocyanates des anhydride acide carboxylique D.

Le procédé de synthèse des (co)polymères en voie solvant est un procédé tel que :
- on fait réagir à une température allant de 20 à 200°C, préférentiellement de 50 à 150°C, dans un solvant aprotique polaire au moins un composé A comprenant deux fonctions anhydride (ou au moins un composé D comprenant une fonction anhydride et une fonction acide carboxylique) avec un diisocyanate B', et éventuellement en présence d'au moins un diisocyanate C', lesdits composés A (ou D), B' et C' étant tels que définis précédemment, pour former directement en une seule étape le polyimide (ou polyamideimide). Le temps de réaction peut être compris entre 5 min et 100 heures, préférentiellement entre 10 min et 10 heures. La réaction est suivie par le dégagement de CO2.

### Synthèse par voie fondu

Les synthèses par voie fondu impliquent que les monomères ou précurseurs sont portés à une température :
- supérieure à la température de fusion du (co)polymère si le (co)polymère est semi-cristallin ou
- supérieure à la température de transition vitreuse si le (co) polymère est amorphe.

La polymérisation à l'état fondu peut être effectuée à partir :
o d'une diamine (B + éventuellement C) ou d'un diisocyanate (B' + éventuellement C') et d'un dianhydride ou de ses dérivés tétraacide ou diester ou triester ou tétraester (composé A), ou d'un composé D
o d'un sel de diamine et d'un tétraacide ou d'un diester

Avantageusement, la polymérisation en voie fondu est effectuée à partir des sels qui présente l'avantage de contrôler finement la stoechiométrie. Dans le cas des sels, on peut ajouter une quantité d'eau permettant la solubilisation des sels ou leur dispersion.

La réaction peut être conduite dans un réacteur de synthèse ou dans une extrudeuse munie de système de dégazage des vapeurs.

Des polymérisations à l'état fondu sont notamment décrites le brevet US2710853 à partir de diamine aliphatique et d'anhydride pyromellitique ou de dérivés diester diacide d'anhydride pyromellitique.

Le contrôle de la masse molaire moyenne en nombre peut être obtenu :
- par l'utilisation de limiteurs de chaînes, c'est-à-dire des molécules choisies parmi les monoamines, les monoanhydrides, les monoacides ou les diacides en positions α,β tels qu'ils peuvent former une fonction anhydride par réaction de déshydratation, parmi les limiteurs de chaîne on peut citer l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,
- par un déséquilibre stoechiométrique r=[composés A]/[diamines B + C ou diisocyanates B' + C'], ou r=[composés D]/[diamines B + C ou diisocyanates B' + C']
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à tout moment du procédé de fabrication.

En particulier le déséquilibre stoechiométrique r peut aller de 1,01 à 1,2. Cette plage est avantageuse car elle permet d'éviter la formation de gels par réticulation de l'amine.

La teneur en limiteur de chaîne peut aller de 0,1 à 10 % en nombre de moles, notamment de 1 à 5% en nombre de moles, par rapport au nombre total de moles de monomères, c'est à dire acide/dianhydride tétracarboxylique (ou anhydride acide dans le cas du composé D), diamine/diisocyanate (B et éventuellement C ou B' et éventuellement C') et limiteur de chaine.

Dans ce procédé, des catalyseurs, des charges inorganiques inertes ou réactives (argiles, silices ou précurseurs de silice, nanoparticules, ...), des stabilisants, des mâtifiants ou des colorants peuvent également être introduits.

### Synthèse par voie solide, à partir des acides tétracarboxyliques et diamines ou acides tricarboxyliques et diamines.

Le principe de la synthèse en voie solide consiste à préparer le (co)polymère à une température inférieure à la température de fusion du (co)polymère à partir d'un précurseur ; T < Tf(PI) dans le cas d'un (co)polymère semi-cristallin ou à T < Tg(PI) dans le cas d'un (co)polymère amorphe.
Il vient d'être mis en évidence par la demanderesse une nouvelle voie de préparation industrielle et efficace des (co)polymères.
Cette synthèse est rendue possible par l'utilisation d'une polymérisation à l'état solide d'un sel solide de carboxylate d'ammonium formé à partir d'une diamine B et d'un acide tétracarboxylique A ou d'un sel solide de carboxylate d'ammonium formé à partir d'une diamine B et d'un acide tricarboxylique D . Le procédé de l'invention permet l'obtention de poudres de granulométries contrôlées puisque la réaction de polymérisation se produit à l'état solide.

Par ailleurs, la polymérisation à l'état solide permet d'éviter l'utilisation de solvants cancérigènes ou néfastes pour l'environnement.

Un autre avantage du procédé de l'invention est la faculté d'effectuer une polymérisation à une température relativement faible permettant d'éviter des dégradations thermiques du sel et du (co)polymère formé.

La présente invention concerne ainsi un procédé de préparation de (co)polyimides selon l'invention comprenant au moins les étapes suivantes :
(a) on place dans un réacteur un sel formé par une réaction entre au moins une diamine B et au moins un acide tétracarboxylique A ou au moins une diamine B et au moins un acide tricarboxylique D ;
(b) on effectue une polymérisation à l'état solide à partir du sel de l'étape (a) pour obtenir le (co)polymère à une pression absolue comprise entre 0,005 et 1 MPa et à une température T obéissant à la relation suivante, pour un (co)polymère semi-cristallin :
   Tf du (co)polymère à obtenir > T
   préférentiellement
   Tf du (co)polymère à obtenir > T > Tg du (co)polymère à obtenir
   et encore plus préférentiellement
   Tf du sel de l'étape (a) > T > Tg du (co)polymère à obtenir,
   Ou à une température T obéissant à la relation T<Tg pour un (co)polymère amorphe
   et
(c) on récupère les particules solides (co)polymère.

### Etape (a)

Lors de l'étape (a) du procédé, on place ainsi dans un réacteur un sel formé par une réaction entre au moins une diamine B +/- C et au moins un acide tétracarboxylique A ou au moins une diamine B +/- C et au moins un acide tricarboxylique D.

Un tel sel peut être synthétisé de diverses manières, connues de l'homme du métier.

On peut par exemple procéder à une addition d'une diamine B +/- C dans une solution comprenant l'acide tétracarboxylique A (ou comprenant l'acide tricarboxylique D). On peut également dissoudre l'acide tétracarboxylique A (ou l'acide tricarboxylique D) dans un solvant tel que de l'alcool, comme l'éthanol ou du méthanol par exemple, et faire de même pour la diamine B +/- C. Ces deux solutions sont alors mélangées sous agitation. Le sel de carboxylate d'ammonium formé peut être insoluble dans le solvant utilisé et ainsi précipiter. Le sel peut alors être récupéré par filtration, lavé et séché et éventuellement broyé.

On peut également réaliser une solution de sel de carboxylate d'ammonium puis la concentrer à chaud et ensuite la refroidir. Le sel cristallise alors et les cristaux sont récupérés et séchés. La concentration de la solution peut être obtenue par évaporation du solvant comme l'eau ou l'alcool ou selon un autre procédé par addition d'acide tétracarboxylique A (ou l'acide tricarboxylique D) et/ou de diamine B +/- C. On peut également procéder à une saturation de la solution, c'est-à-dire effectuer un procédé qui permet de modifier la concentration du sel dans la solution à une valeur compatible avec une cristallisation de celui-ci. Généralement cette concentration est au moins égale et plus préférentiellement supérieure à la concentration de saturation du sel à la température considérée. Plus précisément, cette concentration correspond à une sursaturation de la solution du sel. On peut également travailler à une pression permettant d'évaporer le solvant de la solution, tel que l'eau ou l'alcool, pour saturer la solution et provoquer la cristallisation. On peut aussi saturer la solution par addition successive ou simultanée d'un flux d'acide tétracarboxylique A (ou l'acide tricarboxylique D) et d'un flux de diamine B +/- C dans une solution de sel.

A titre d'exemple, on dissout l'acide tétracarboxylique dans de l'alcool, comme l'éthanol par exemple, dans un premier milieu. On dissout la diamine B +/- C dans de l'alcool dans un autre milieu et on mélange ensuite les deux milieux sous agitation. Le sel obtenu précipite.

A la fin de cette synthèse le sel peut être sous forme de poudre sèche, sous forme de poudre dispersée dans un solvant, ou dissout en solution. On peut récupérer le sel par filtration dans le cas d'un précipitât et désagréger le gâteau de filtration si nécessaire. Dans le cas ou le sel est dissout en solution, on peut le récupérer par un procédé de cristallisation par concentration, sursaturation ou en le faisant précipiter par addition d'un non solvant. Le sel cristallisé peut alors être récupéré par filtration et le gâteau de filtration peut être désagrégé si nécessaire. Un autre procédé permettant de récupérer les particules dispersées de sel sec est l'atomisation de la solution, c'est-à-dire notamment une opération d'évaporation soudaine du solvant pulvérisé sous forme de fines gouttelettes afin de récupérer les particules dispersées de sel.

Il est enfin possible de cribler la dimension des particules de sel, par exemple par tamisage ou broyage.

### Etape (b)

Lors de l'étape (b) du procédé, on effectue ainsi une polymérisation à l'état solide à partir du sel de l'étape (a) pour obtenir le (co)polymère à une pression absolue comprise entre 0,005 et 1 MPa et à une température T obéissant à la relation telle que décrite précédemment.

La pression absolue lors de l'étape (b) est préférentiellement comprise entre 0,005 MPa et 0,2 MPa.

La température lors de l'étape (b) est préférentiellement comprise entre 100°C et 300°C.

Le procédé de polymérisation à l'état solide peut être réalisé selon les procédés classiques connus de l'homme du métier. Le principe fondamental de ces procédés consiste à porter le sel de départ, sous air ou dans une atmosphère inerte ou sous vide, à une température inférieure à son point de fusion mais suffisante pour permettre la réaction de polymérisation, généralement supérieure à la température de transition vitreuse du (co)polymère. Un tel procédé peut donc comprendre brièvement :
a) un chauffage du produit par diffusion conductive, convective ou par radiation,
b) un inertage par application de vide, balayage par un gaz neutre tel que l'azote, le CO₂, ou la vapeur surchauffée, ou application d'une surpression,
c) une élimination du sous-produit de condensation par évaporation puis, balayage du gaz vecteur ou concentration de la phase gaz,
d) une agitation mécanique ou fluidisation de la phase solide par le gaz vecteur ou vibrations peut être souhaitable afin d'améliorer les transferts thermiques et massiques et également prévenir tout risque d'agglomération du solide divisé.

Préférentiellement, on utilise dans l'étape b) un moyen de maintien en mouvement des particules de sel de (co)polymères afin de prévenir une agrégation de ces particules. On peut utiliser pour ce faire une agitation mécanique, tel qu'un agitateur, une mise en rotation du réacteur, ou une agitation par vibrations, ou une fluidification par un gaz vecteur.

La masse molaire moyenne en nombre Mn des (co)polymères peut être comprise entre 1000 g/mol à 100000 g/mol.

Le contrôle de la masse molaire moyenne en nombre peut être obtenu :
- par l'utilisation de limiteurs de chaînes, c'est-à-dire des molécules choisies parmi les monoamines, les monoanhydrides les monoacides ou les diacides en positions α,β tels qu'ils peuvent former une fonction anhydride par réaction de déshydratation. Des exemples de limiteurs de chaîne sont l'anhydride phtalique, le 1-aminopentane, le 1-aminohexane, le 1-aminoheptane, le 1-aminooctane, le 1-aminononane, le 1-aminodécane, le 1-aminoundécane, le 1-aminododécane, la benzylamine, l'acide ortho-phtalique (ou acide 1,2-benzènedicarboxylique), l'acide acétique, l'acide propionique, l'acide benzoïque, l'acide stéarique ou leurs mélanges,

- par un déséquilibre stoechiométrique r=[composés A]/[diamines B +/- C] ou R=[composés D]/[diamine B +/- C]
- par l'utilisation d'agents de branchement, c'est-à-dire des molécules de fonctionnalité supérieure à 3,
- par l'ajustement des conditions opératoires de synthèses telles que le temps de séjour, la température, l'humidité ou la pression, ou
- par une combinaison de ces différents moyens.

Le contrôle de la stoechiométrie peut être fait à n'importe quel moment du procédé de fabrication.

En particulier le déséquilibre stoechiométrique r peut aller de 1,01 à 1,2.

Selon un mode de réalisation particulier, le sel est :
- additionné de limiteurs de chaînes et/ou
- additionné d'un excès d'un des monomères, afin de créer un déséquilibre stoechiométrique, c'est-à-dire afin que r soit différent de 1.

Selon une variante, le limiteur de chaîne est additionné au sel de l'étape (a) déjà formé.

Selon une autre variante, le limiteur de chaîne est également sous forme de sel, en particulier il forme un sel avec la diamine B +/- C et/ou avec l'acide tétracarboxylique A. (ou l'acide tricarboxylique D) En particulier le limiteur de chaine est présent lors de la formation du sel de l'étape (a) et est additionné en même temps que l'espèce lui correspondant, par exemple limiteur de type acide avec l'acide tétracarboxylique A et limiteur de type amine avec l'amine B +/- C.
Dans ce deuxième cas, le limiteur de chaîne permet la formation de sel, et notamment peut être choisi dans les listes ci-dessus, à l'exception des anhydrides.

La teneur en limiteur de chaîne peut aller de 0,1 à 10 % en nombre de moles, notamment de 1 à 5% en nombre de moles, par rapport au nombre total de moles de monomères, c'est à dire acide tétracarboxylique A (ou l'acide tricarboxylique D) diamine B +/- C et limiteur de chaine.

Lorsqu'un limiteur de chaine est utilisé, les quantités d'amines et d'acides peuvent être équilibrées, c'est-à-dire que la somme de fonctions amines est sensiblement égale à la moitié de la somme de fonctions acides avec lesquelles elles peuvent réagir. Par « sensiblement égale » on entend une différence maximum de 1 %.

Lorsqu'un limiteur de chaine est utilisé, les quantités d'amines et d'acides peuvent être déséquilibrées, c'est-à-dire que la somme de fonctions amines est sensiblement différente à la moitié de la somme de fonctions acides avec lesquelles elles peuvent réagir. Par « sensiblement différente » on entend une différence d'au moins 1 %.

Des catalyseurs peuvent également être introduits, et également des charges inorganiques inertes ou réactives (argiles, silices ou précurseurs de silice, nanoparticules, ...), des stabilisants, des mâtifiants, des colorants, etc.

On peut utiliser des catalyseurs, ajoutés à n'importe quel moment du procédé, tel que par exemple en mélange avec la diamine et/ou l'acide tétracarboxylique, en mélange au sel formé soit en solution soit par imprégnation à l'état solide.

Par ailleurs, il existe des applications pour lesquelles il est nécessaire que les polymères se présentent sous la forme de poudres. C'est le cas notamment du frittage laser ou des procédés de fabrication de composites à fibres continues à partir de poudres par poudrage d'étoffes ou pultrusion de monofil de verre ou de carbone, ou encore d'autres procédés. Les technologies de production de poudres de polymères connues nécessitent soit de dissoudre un polymère dans un solvant et de précipiter dans un non solvant ; mais ceci implique l'utilisation de solvants toxiques et cancérigènes, soit de mélanger à l'état fondu le polymère avec une espèce non miscible de sorte à générer une ségrégation du polymère souhaitée, soit de broyer des granulés de polymères formulés ce qui impose des étapes de micronisation et de séchages supplémentaires. Quel que soit le cas cité, les procédés sont complexes et coûteux.

### Compositions

### Cas des (co)polymères thermoplastiques :

On peut utiliser le (co)polymère de l'invention pour faire de compositions qui sont généralement obtenues par mélange des différents composés, charges et/ou additifs. On procède à plus ou moins haute température, à plus ou moins haute force de cisaillement selon la nature des différents composés. Les composés peuvent être introduits simultanément ou successivement. On utilise généralement un dispositif de mélange d'extrusion dans lequel la matière est chauffée, puis fondue et soumise à une force de cisaillement, et véhiculée. On peut, selon des modes de réalisations particuliers, effectuer des pré-mélanges, en fondu ou non, avant préparation de la composition finale. On peut par exemple effectuer un pré-mélange dans une résine, par exemple du (co)polymère, de façon à réaliser un mélange maitre.

L'invention concerne ainsi également un procédé de fabrication d'une composition à base de (co)polymères selon l'invention, par mélange en fondu ou non dudit (co)polymère avec des charges de renfort ou de remplissage et/ou des agents modificateurs du choc et/ou des additifs.

La composition selon l'invention peut éventuellement comprendre un ou plusieurs autres polymères tels que par exemple les polyamides, les polyesters ou les polyoléfines. Ces autres polymères représentent avantageusement moins de 40% en poids par rapport au poids de la composition.

La composition selon l'invention peut comprendre entre 20 et 90 % en poids, préférentiellement entre 20 et 70 % en poids, et plus préférentiellement entre 35 et 65% en poids de (co)polymères selon l'invention obtenu par le procédé de polymérisation tel que décrit précédemment, par rapport au poids total de la composition.

La composition peut en outre comprendre des charges de renfort ou de remplissage. Les charges de renfort ou de remplissage sont des charges classiquement utilisées pour la réalisation de compositions thermoplastiques, notamment à base de polyamide. On peut notamment citer les charges fibreuses de renfort, telles que telles que des fibres de verre, des fibres de carbone, ou des fibres organiques, les charges non fibreuses, telles que des charges particulaires, lamellaires et/ou les nanocharges exfoliables ou non exfoliables comme l'alumine, le noir de carbone, les argiles, le phosphate de zirconium, le kaolin, le carbonate de calcium, le cuivre, les diatomées, le graphite, le mica, la silice, le dioxyde de titane, les zéolites, le talc, la wollastonite, les charges polymériques telles que, par exemple, des particules de diméthacrylates, les billes de verre ou de la poudre de verre. On préfère notamment utiliser les fibres de renfort, telles que les fibres de verre.

La composition selon l'invention peut comprendre entre 5 et 60 % en poids de charges de renfort ou de remplissage, préférentiellement entre 10 et 40 % en poids, par rapport au poids total de la composition.

La composition selon l'invention comprenant le (co)polymère tel que défini précédemment peut comprendre au moins un agent modificateur du choc, c'est-à-dire un composé capable de modifier la résistance aux chocs d'une composition (co)polymère. Ces composés modificateurs du choc comprennent préférentiellement des groupements fonctionnels réactifs avec le (co)polymère. On entend selon l'invention par groupements fonctionnels réactifs avec le (co)polymère, des groupements capables de réagir ou d'interagir chimiquement avec les fonctions résiduelles anhydride, acide ou amine du (co)polymère, notamment par covalence, interaction ionique ou hydrogène ou liaison de van der Walls. De tels groupements réactifs permettent d'assurer une bonne dispersion des agents modificateurs de chocs dans la matrice (co)polymère. On peut citer par exemple les fonctions anhydrides, époxydes, esters, amines, acides carboxyliques, les dérivés carboxylates ou sulfonates.

La composition selon l'invention peut en outre comprendre des additifs généralement utilisés pour la fabrication de compositions polyimides ou polyamides. Ainsi, on peut citer les lubrifiants, les agents ignifugeants, les plastifiants, les agents nucléants, les agents anti-UV, les catalyseurs, les antioxydants, les antistatiques, les colorants, les matifiants, les additifs d'aide au moulage ou autres additifs conventionnels.

Ces charges, agents de renfort de chocs et/ou additifs peuvent être ajoutés au (co)polymères par des moyens usuels adaptés biens connus dans le domaine des plastiques techniques, tel que par exemple lors de la salification, après la salification, lors de la polymérisation à l'état solide, ou en mélange en fondu.

Les compositions (co)polymères sont généralement obtenues par mélange des différents composés entrant dans la composition à froid ou en fondu. On procède à plus ou moins haute température, à plus ou moins haute force de cisaillement selon la nature des différents composés. Les composés peuvent être introduits simultanément ou successivement. On utilise généralement un dispositif d'extrusion dans lequel la matière est chauffée, puis fondue et soumise à une force de cisaillement, et véhiculée.

On peut mélanger tous les composés en phase fondue au cours d'une unique opération, par exemple au cours d'une opération d'extrusion. On peut par exemple procéder à un mélange de granulés des matériaux polymériques, les introduire dans le dispositif d'extrusion afin de les fondre et de les soumettre à un cisaillement plus ou moins important. On peut, selon des modes de réalisations particuliers, effectuer des pré-mélanges, en fondu ou non, de certains des composés avant préparation de la composition finale.

### Cas des (co)polymères non-thermoplastiques (passage par polyacide amique)

Dans le cas des (co)polymères selon l'invention qui ne sont pas thermoplastiques et ne peuvent donc pas être fondus pour être transformés en articles, des charges peuvent être ajoutées au polyacide amique solide, en solution ou dispersé dans un solvant. Ces charges peuvent être des charges de renfort ou de remplissage et/ou des additifs tels que cités ci-dessus.

### Applications

### Cas des (co)polymères thermoplastiques :

Le (co)polymère ou les diverses compositions selon l'invention peuvent être utilisées pour tout procédé de mise en forme de fabrication d'articles.

L'invention concerne ainsi également un procédé de fabrication d'article mettant en oeuvre le (co)polymère selon l'invention. On peut citer à cette effet diverses techniques telles que le procédé de moulage, notamment le moulage par injection, l'extrusion, l'extrusion soufflage, ou encore le rotomoulage, notamment dans le domaine de l'automobile, de l'électronique, l'aéronautique et de l'électricité par exemple. Le procédé d'extrusion peut notamment être un procédé de filage ou de fabrication de films.

La présente invention concerne par exemple la fabrication d'articles de type étoffes imprégnées ou articles composites à fibres continues. Ces articles peuvent notamment être fabriqués par mise en présence d'une étoffe et des particules de (co)polymère selon l'invention à l'état solide ou fondu. Les étoffes sont des surfaces textiles obtenues par assemblage de fils ou de fibres solidarisés par un procédé quelconque, tel que notamment collage, feutrage, tressage, tissage, tricotage. Ces étoffes sont aussi désignées comme des réseaux fibreux ou filamenteux, par exemple à base de fibres de verre, de fibres de carbone ou autres. Leur structure peut être aléatoire, unidirectionnelle (1 D), ou multidirectionnelle (2D, 2,5D, 3D ou autre).

Les particules de (co)polymères de l'invention peuvent notamment être utilisées dans des procédés de fabrication d'articles par fusion sélective de couches de poudre de polymères, notamment le prototypage rapide par frittage en phase solide à l'aide d'un laser. La fabrication par fusion sélective de couches est un procédé de fabrication d'articles consistant à déposer des couches de matériaux sous forme de poudre, à fondre de manière sélective une partie ou un domaine d'une couche, et de venir déposer une nouvelle couche de poudre et de fondre de nouveau une partie de cette couche et ainsi de suite de manière à obtenir l'objet désiré. La sélectivité de la partie de la couche à fondre est obtenue par exemple grâce à l'utilisation d'absorbeurs, d'inhibiteurs, de masques, ou à travers l'apport d'énergie focalisée, comme par exemple un rayonnement électromagnétique tel qu'un rayon laser. On préfère notamment le frittage par additivation de couches, particulièrement le prototypage rapide par frittage à l'aide d'un laser.

### Cas des (co)polymères non thermoplastiques (passage par polyacide amique :

Dans le cas des (co)polymères selon l'invention qui ne sont pas thermoplastiques et ne peuvent donc pas être fondus pour être transformés en articles, les applications potentielles sont la réalisation de films, de revêtements (« coatings »), notamment dans le domaine de l'électronique, de pièces moulées, de matériaux composites en association avec des fibres de verre ou de carbone. Il peut aussi s'agit de fibres ou filaments obtenus par un procédé par exemple tel que décrit dans US4460526, comprenant une étape de dissolution du polyacide amique dans un solvant organique polaire, ladite solution étant ensuite soumise à une étape de filage.

### Propriétés

Les (co)polymères de l'invention présentent l'avantage d'être biosourcés, d'avoir une Tg très élevée, d'être très résistants, de pouvoir être semi-cristallins et de pouvoir apporter une hydrophilie. Il peuvent pour certains être thermoplastiques et présenter l'avantage d'être transformable en fondu. Ils présentent également une bonne résistance aux solvants de type alcool, xylène, NMP.

Le terme « et/ou » inclut les significations « et », « ou », ainsi que toutes les autres combinaisons possibles des éléments connectés à ce terme.

D'autres détails ou avantages de l'invention apparaîtront plus clairement au vu des exemples donnés ci-dessous uniquement à titre indicatif.

### PARTIE EXPERIMENTALE

### Normes de mesures :

Les températures de fusion (Tf) et de cristallisation au refroidissement (Tc) des (co)polymères sont déterminées par calorimétrie différentielle à balayage (DSC « Differential Scanning Calorimetry »), à l'aide d'un appareil Perkin Elmer Pyris 1, à une vitesse de 10°C/min. Les Tf et Tc des (co)polymères sont déterminées au sommet des pics de fusion et de cristallisation. La température de transition vitreuse (Tg) déterminée sur le même appareil à une vitesse de 40 °C/min (lorsque cela est possible, elle est déterminée à 10°C/min et spécifiée dans les exemples). Les mesures sont faîtes après fusion du (co)polymère formé à T>(Tf du (co)polymère + 20°C).

Lorsque l'on synthétise des polyimides à partir de sels, la température de fusion du sel est déterminée comme la température de fin de l'endotherme mesurée par chauffage du sel à 10°C/min.

L'Analyse Thermo-Gravimétrique (ATG) est réalisée sur un appareil Perkin-Elmer TGA7 sur un échantillon d'environ 10 mg, par chauffage à 10°C/min sous balayage d'azote jusqu'à 600°C.

L'analyse RMN du proton est effectuée sur un spectromètre Brüker AV500.

### Exemple 1 : synthèse du PI TFPMA

La diamine 2,5-bis(aminométhyl)tétrahydrofurane (TF) cis/trans 90/10 est synthétisée selon le mode opératoire suivant :
- La molécule de départ le tétrahydrofurane-2,5-diméthanol, est synthétisé à partir de la réaction dans le méthanol du 5-(Hydroxyméthyl)furfural (HMF) avec le nickel de Raney (1,5 équivalent par rapport à l'HMF) sous pression de 5,84 bars d'H₂ à 60°C pendant 20 heures, filtration et purification par distillation. Un liquide légèrement jaune est obtenu de pureté supérieure à 98% (déterminé par chromatographie / couplée spectroscopie de masse). Rendement de la réaction : 95%.
- Le chlorure de méthanesulfonyle (307,8 g, 2,7 mol) est ajouté goutte à goutte à une solution de tétrahydrofurane-2,5-diméthanol (118,8 g, 900 mmol) et de triéthylamine (454,5g, 4500 mmol) dans 1,54 L de dichlorométhane à 0 °C. Le milieu réactionnel est maintenu à 0 °C pendant 1 heure puis ajouté à de l'eau glacée et la phase organique est séparée et lavée avec 500 mL d'une solution d'acide chlorhydrique diluée (1 M). La phase organique est séparée puis lavée avec 500 mL d'une solution aqueuse saturée en NaHCO₃. La phase organique est enfin séparée et concentrée pour donner 236,7 g de (tétrahydrofuran-2,5-diyl)bis(méthylène) diméthanesulfonate sous la forme d'une huile brune de pureté égale à 96% (déterminée par LCMS). Rendement de la réaction : 91,0%. Une solution de (tétrahydrofuran-2,5-diyl)bis(méthylène) diméthanesulfonate (236,7 g, 821,7 mmol) et NaN₃ (270,0g, 4,1094 mol) dans le DMSO (1,350 L) est chauffée à 95°C et agitée une nuit. Le milieu réactionnel est ajouté à de l'eau glacée et extrait avec 3 fois 700 mL d'acétate d'éthyle. Les extraits (phase contenant l'acétate d'éthyle) sont successivement lavés avec de l'eau, une solution aqueuse saturée en NaHCO₃ et séchée une nuit sur MgSO₄ puis filtré pour retirer le MgSO₄. La phase contenant l'acétate d'éthyle est concentrée pour donner 166,5 g de 2,5-bis(azidométhyl)tétrahydrofurane sous la forme d'une huile brune. Un mélange de 2,5-bis(azidométhyl)tétrahydrofurane (166,5 g) et de Pd-C (10%, 10,8 g) dans le méthanol (2,7L) est agité une nuit à température ambiante sous 1 atm de H₂. Le milieu réactionnel est filtré et le filtrat est concentré sous vide pour donner 90,0g de 2,5-bis(aminométhyl)tétrahydrofurane sous la forme d'une huile jaune. Le rendement total des 3 réactions successives est de 75%. Le procédé de synthèse du 2,5-bis(aminométhyl)tétrahydrofurane donne un mélange des isomères cis/trans 90/10 d'après l'analyse en RMN C¹³ dans le méthanol deutéré.

On prépare dans un flacon une solution méthanolique (solution 1) en dissolvant 1,78g (0,0137 mol) de 2,5-bis(aminométhyl)tétrahydrofurane dans 15 g de méthanol à température ambiante. Dans un ballon de 100 mL à température ambiante, on introduit 20 g de méthanol et 3,39 g (0,0133) d'acide pyromellitique (Solution 2) sous agitation. Lorsque tout l'acide pyromellitique est dissout, on introduit sous agitation en 2 minutes la solution 1 dans la solution 2. Le flacon vide de la solution 1 est rincé par 10g de méthanol et rajouté dans le ballon. Le milieu réactionnel est agité à température ambiante pendant 2 heures.
Le précipité formé (sel TFPMA) est récupéré par filtration, lavé au méthanol et séché. L'analyse RMN du proton dans le diméthyle sulfoxide montre que le sel est stoechiométrique.

Le sel est placé dans un tube en verre inerté par un flux d'azote et chauffé à 210°C pendant 5 heures pour effectuer la polymérisation. La poudre de sel se transforme en poudre de polyimide PI TFPMA avec dégagement d'eau.

L'analyse thermique en DSC (première chauffe à 10°C/min) jusqu'à 300°C montre un phénomène exothermique avec un pic à 263°C associé à une cristallisation. Après 1 minute à 300°C, l'échantillon est refroidi à 10°C/min jusqu'à 20°C, maintenu 1 min à 20°C et chauffé à 10°C/min jusqu'à 300°C. On observe ainsi une température de transition vitreuse à Tg à 256°C mais la fusion n'est pas observée (supérieure à 300°C)..

Le PI TFPMA est donc un polyimide semi-cristallin, de Tf>300°C, et de Tg=256°C. Il possède une Tg du même ordre de grandeur que celle du polyimide PI 1,3-BAMCPMA obtenu à partir d'acide pyromellitique et de la 1,3-bis(aminométhyle)cyclohexane décrit dans le brevet JP2012092262 (Tg=260°C). Les deux polymères sont semi-cristallins et de Tg voisine, le PI TFPMA est donc bien une alternative biosourcée au PI 1,3-BAMCPMA.

### Exemple comparatif : synthèse du PI MXDPMA

On utilise le même mode opératoire que celui de l'exemple 1 pour préparer une poudre de sel MXDPMA à partir de méta-xylylène diamine (1,762g soit 0,0129 mol) et d'acide pyromellitique (3,34 g, 0,0131 mol). La poudre de sel est récupérée par filtration, lavée, séchée et chauffée à 210°C pendant 5 heures pour effectuer la polymérisation.

La Tg du PI MXDPMA mesurée dans les mêmes conditions que celles de l'exemple 1 est détectée à 275°C.

Conclusion : le PI TFPMA présente une alternative bio-sourcée intéressante aux PI MXDPMA et PI 1,3-BAMCPMA.

Ce PI MXDPMA est soluble à température ambiante à 10g/L dans l'acide sulfurique concentré à 96% et insoluble à température ambiante à 10 g/L dans l'acide formique ou le 1,3-diméthylimidazolidinone. Le PI TFPMA présente le même comportement, il présente donc du point de vue à la fois des propriétés thermiques et chimiques une alternative biosourcée à des polyimides issus de ressources fossiles.

## Revendications

1. (Co)polymère comprenant l'unité récurrente de formule I suivante : 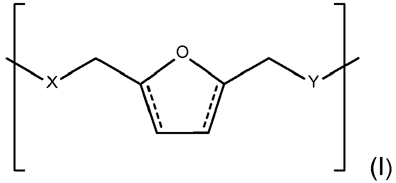 dans laquelle
- X est un groupe divalent de formule la ou Ib suivantes : 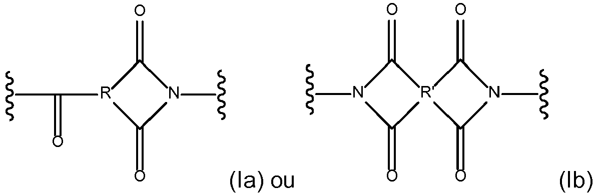 dans lesquelles
• R est un groupe hydrocarboné trivalent choisi parmi:
un groupe aliphatique ou cycloaliphatique, saturé ou insaturé, éventuellement interrompu par un ou plusieurs hétéroatomes, éventuellement substitué par un ou plusieurs groupements fonctionnels ou non ;
un groupe aromatique, alkylaromatique ou arylaliphatique, éventuellement interrompu par un ou plusieurs hétéroatomes, éventuellement substitué par un ou plusieurs groupements fonctionnels ou non ;
• R' est un groupe hydrocarboné tétravalent choisi parmi:
un groupe aliphatique ou cycloaliphatique, saturé ou insaturé, éventuellement interrompu par un ou plusieurs hétéroatomes, éventuellement substitué par un ou plusieurs groupements fonctionnels ou non ;
un groupe aromatique, alkylaromatique ou arylaliphatique, éventuellement interrompu par un ou plusieurs hétéroatomes, éventuellement substitué par un ou plusieurs groupements fonctionnels ou non ;
- Y est :
• une liaison covalente, lorsque X répond à la formule Ib,
• un groupe divalent de formule Ic ou Id suivantes, lorsque X répond à la formule Ia : 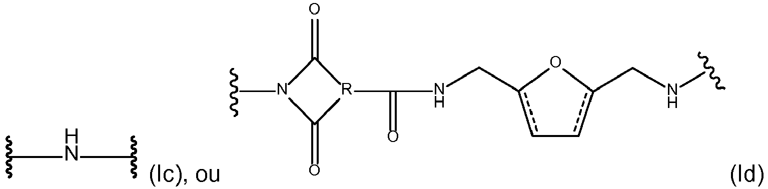 avec R tel que défini ci-dessus.

2. (Co)polymère selon la revendication 1 obtenu par polymérisation des composés suivants :
- au moins un composé A comprenant deux fonctions anhydride et/ou ses équivalents acide tétracarboxylique et/ou ester; et
- au moins une diamine B de formule II ou III, 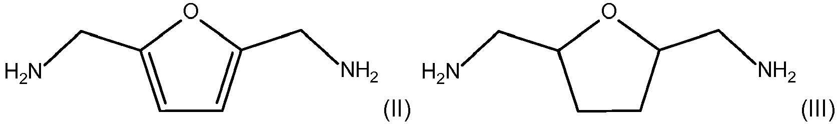 ou
- au moins un diisocyanate B' de formule II' ou III' suivantes : 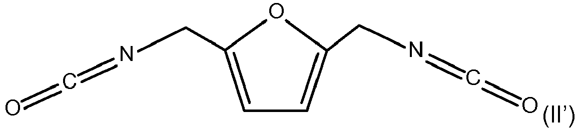 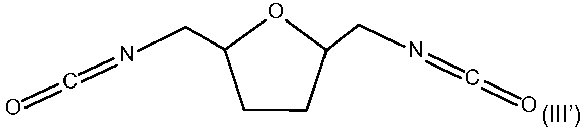
ou
- au moins un sel de carboxylate d'ammonium obtenu à partir des composés A et B.

3. (Co)polymère selon la revendication 2, **caractérisé en ce que** le composé A est l'anhydride pyromellitique ou l'acide pyromellitique.

4. (Co)polymère selon la revendication 1 obtenu par polymérisation des composés suivants :
- au moins un composé D comprenant une fonction anhydride et une fonction acide carboxylique et/ou ses équivalents acide tricarboxylique et/ou ester et/ou anhydride chlorure d'acide ; et
- au moins une diamine B de formule II ou III, 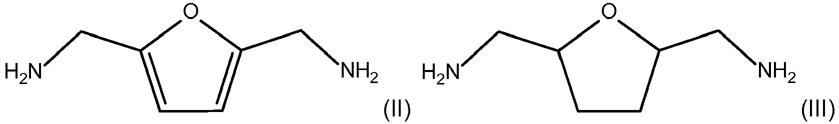 ou
- au moins un diisocyanate B' de formule II' ou III' suivantes : 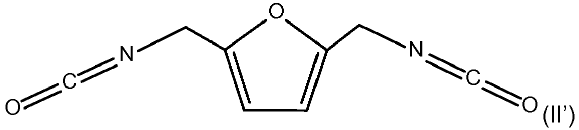 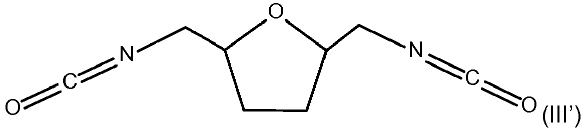
ou
- au moins un sel de carboxylate d'ammonium obtenu à partir des composés D et B.

5. (Co)polymère selon la revendication 4, **caractérisé en ce que** le composé D est choisi parmi:
• l'anhydride trimellitique,
• les triacides carboxyliques choisis parmi : l'acide trimellitique, l'acide tricarballylique, l'acide citrique, l'acide aconitique, l'acide 1,2,4-butane tricarboxylique, l'acide 1,2,3-benzène tricarboxylique,
• les monoester diacides, les diesters monoacide ou triesters desdits triacides carboxyliques,
• le chlorure d'anhydride trimellitique.

6. (Co)polymère selon l'une des revendications 2 à 5, **caractérisé en ce que** la diamine B est une diamine de formule III suivante :

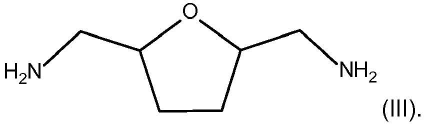

7. (Co)polymère selon l'une quelconque des revendications précédentes, **caractérisé en ce qu'**il est obtenu par polymérisation d'au moins un composé A comprenant deux fonctions anhydride et/ou ses équivalents acide tétracarboxylique et/ou ester ou D comprenant une fonction anhydride et une fonction acide carboxylique et/ou ses équivalents acide tricarboxylique et/ou ester et/ou anhydride chlorure d'acide et d'au moins la diamine B de formule II ou III suivantes : 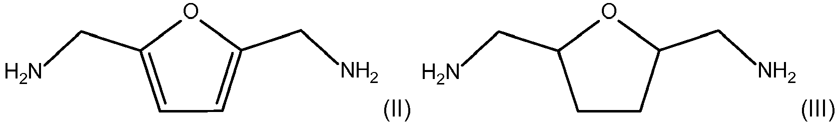 ou d'au moins le diisocyanate B' de formule II' ou III' suivantes : 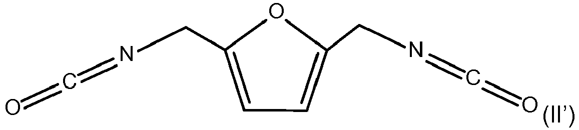 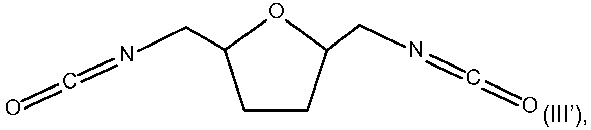 en présence d'au moins une diamine C ou d'au moins un diisocyanate C' de formule IV ou IV' suivantes :
NH₂-R₃-NH₂ (IV)
O=C=N-R₃-N=C=O (IV')
avec R₃ étant un radical divalent hydrocarboné aliphatique, cycloaliphatique ou arylaliphatique, saturé et/ou insaturé, aromatique ou alkylaromatique, qui comprend éventuellement des hétéroatomes.

8. Polyacide amique répondant à la formule V suivante : 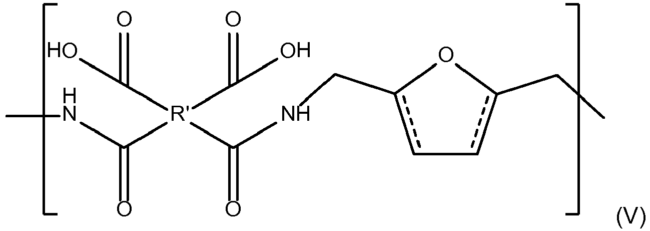 avec R' étant tel que défini à la revendication 1.

9. Procédé de fabrication de (co)polymère tel que défini à l'une des revendications 1 à 7 par polymérisation en solution, à l'état solide ou à l'état fondu, d'au moins un composé A et d'au moins une diamine B éventuellement en présence d'au moins une diamine C, lesdits composés A, B et C étant tels que définis à l'une quelconque des revendications 2 à 7, ou par polymérisation en solution d'un sel de carboxylate d'ammonium obtenu à partir desdits composés A, B et éventuellement C.

10. Procédé de fabrication de (co)polymère selon la revendication 9, **caractérisé en ce qu'**il s'agit d'une polymérisation en solution et qu'il comprend les étapes suivantes :
- on fait réagir entre -20 et 120°C, dans un solvant aprotique polaire ou un solvant phénolique au moins un composé A comprenant deux fonctions anhydride avec une diamine B, et éventuellement en présence d'au moins une diamine C, lesdits composés A, B et C étant tels que définis à l'une des revendications 2 à 7, pour former un intermédiaire polyacide amique de formule V tel que défini à la revendication 8 ;
- on chauffe à une température comprise entre 120°C et 350°C et/ou on effectue une déshydratation chimique ;
- on récupère le (co)polymère, soit par évaporation du solvant, soit par précipitation dans un non solvant.

11. Sel de carboxylate d'ammonium obtenu à partir d'un ou plusieurs composés A ou D et une ou plusieurs diamines B et éventuellement une ou plusieurs diamines C, lesdits composés A, B, C et D étant tels que définis à l'une quelconque des revendications 2 à 7.

12. Composition comprenant le (co)polymère tel que défini à l'une quelconque des revendications 1 à 7 et des charges et/ou additifs.

13. Procédé de fabrication d'article à base de (co)polymère selon l'une quelconque des revendications 1 à 7, par mise en forme par extrusion, moulage ou soufflage dudit (co)polymère sous forme fondue lorsqu'il présente une température de fusion inférieure à 350°C pour un (co)polymère semi-cristallin ou une température de transition vitreuse inférieure à 300°C pour un (co)polymère amorphe.

14. Procédé de fabrication d'article à base de (co)polymère selon l'une quelconque des revendications 1 à 7, par cyclisation d'un polyacide amique tel que défini à la revendication 8, sous forme solide ou d'une solution ou d'une dispersion dans un solvant.

15. Article obtenu à partir du (co)polymère tel que défini à l'une quelconque des revendications 1 à 7 ou de la composition telle que définie à la revendication 12.

16. Article selon la revendication 15, **caractérisé en ce qu'**il s'agit d'une pièce moulée, extrudée ou soufflée.

17. Article selon la revendications 16, **caractérisé en ce qu'**il s'agit d'une pièce moulée par injection, d'un composite à fibres continues, d'un film, d'une fibre, d'un fil ou d'un filament, d'une mousse ou d'une pièce tissée ou tricotée à partir desdits fibres, fils ou filaments.

## Patentansprüche

1. (Co)Polymer, umfassend die Wiederholungseinheit der folgenden Formel I: 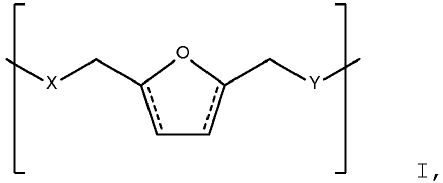 worin
- X eine zweiwertige Gruppe der folgenden Formel Ia oder Ib ist: 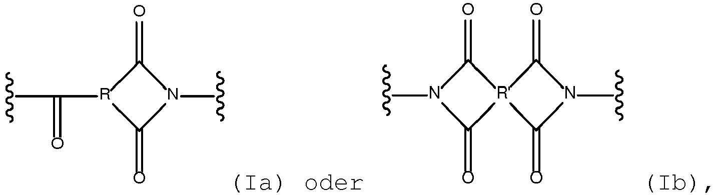 in denen
• R eine dreiwertige Kohlenwasserstoffgruppe ist, ausgewählt aus:
einer gesättigten oder ungesättigten, aliphatischen oder cycloaliphatischen Gruppe, die gegebenenfalls durch ein oder mehrere Heteroatome unterbrochen, gegebenenfalls mit einer oder mehreren funktionellen Gruppen substituiert oder nicht substituiert ist;
einer aromatischen, alkylaromatischen oder arylaliphatischen Gruppe, die gegebenenfalls durch ein oder mehrere Heteroatome unterbrochen, gegebenenfalls mit einer oder mehreren funktionellen Gruppen substituiert oder nicht substituiert ist;
• R' eine vierwertige Kohlenwasserstoffgruppe ist, ausgewählt aus:
einer gesättigten oder ungesättigten, aliphatischen oder cycloaliphatischen Gruppe, die gegebenenfalls durch ein oder mehrere Heteroatome unterbrochen, gegebenenfalls mit einer oder mehreren funktionellen Gruppen substituiert oder nicht substituiert ist;
einer aromatischen, alkylaromatischen oder arylaliphatischen Gruppe, die gegebenenfalls durch ein oder mehrere Heteroatome unterbrochen, gegebenenfalls mit einer oder mehreren funktionellen Gruppen substituiert oder nicht substituiert ist;
- Y Folgendes ist:
• eine kovalente Bindung, wenn X der Formel Ib entspricht,
• eine zweiwertige Gruppe der folgenden Formel Ic oder Id, wenn X der Formel Ia entspricht: 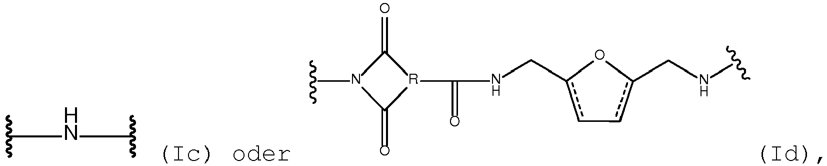 wobei R wie vorstehend definiert ist.

2. (Co) Polymer nach Anspruch 1, das durch Polymerisation der folgenden Verbindungen erhalten wird:
- mindestens einer Verbindung A, die zwei Anhydridfunktionen umfasst, und/oder deren Tetracarbonsäure- und/oder Esteräquivalenten und
- mindestens eines Diamins B der Formel II oder III 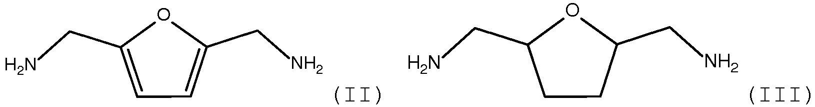 oder
- mindestens eines Diisocyanats B' der folgenden Formel II' oder III': 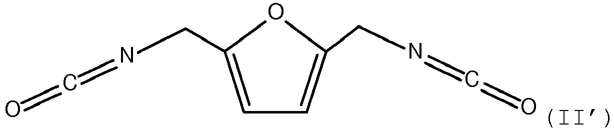 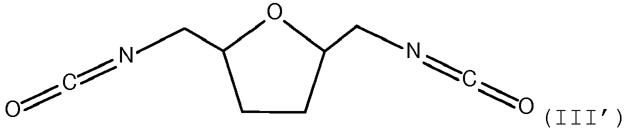 oder
- mindestens eines Ammoniumcarboxylatsalzes, das aus den Verbindungen A und B erhalten wird.

3. (Co)Polymer nach Anspruch 2, **dadurch gekennzeichnet, dass** die Verbindung A Pyromellitsäureanhydrid oder Pyromellitsäure ist.

4. (Co) Polymer nach Anspruch 1, das durch Polymerisation der folgenden Verbindungen erhalten wird:
- mindestens einer Verbindung D, die eine Anhydridfunktion und eine Carbonsäurefunktion umfasst, und/oder deren Tricarbonsäure- und/oder Ester- und/oder Säurechloridanhydridäquivalente und
- mindestens eines Diamins B der Formel II oder III 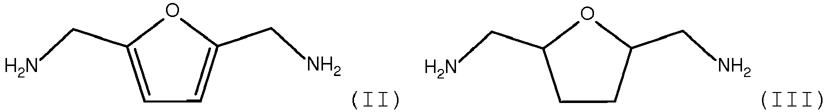 oder
- mindestens eines Diisocyanats B' der folgenden Formel II' oder III': 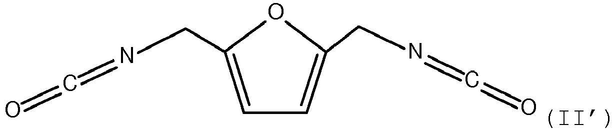 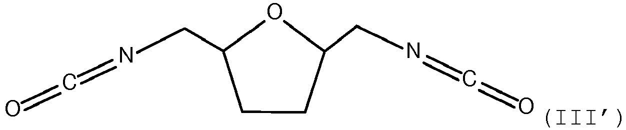 oder
- mindestens eines Ammoniumcarboxylatsalzes, das aus den Verbindungen D und B erhalten wird.

5. (Co)Polymer nach Anspruch 4, **dadurch gekennzeichnet, dass** die Verbindung D aus den Folgenden ausgewählt ist:
• Trimellitsäureanhydrid,
• Tricarbonsäuren ausgewählt aus: Trimellitsäure, Tricarballylsäure, Zitronensäure, Aconitsäure, 1,2,4-Butantricarbonsäure, 1,2,3-Benzoltricarbonsäure,
• Disäuremonoestern, Monosäurediestern oder Triestern dieser Tricarbonsäuren,
• Trimellitsäureanhydridchlorid.

6. (Co)Polymer nach einem der Ansprüche 2 bis 5, **dadurch gekennzeichnet, dass** das Diamin B ein Diamin der folgenden Formel III ist:

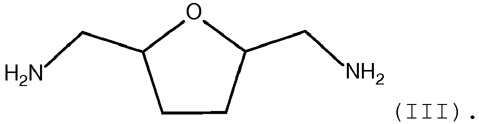

7. (Co)Polymer nach einem der vorhergehenden Ansprüche, **dadurch gekennzeichnet, dass** es erhalten wird durch Polymerisation von mindestens einer Verbindung A, die zwei Anhydridfunktionen umfasst, und/oder deren Tetracarbonsäure- und/oder Esteräquivalenten oder D, die eine Anhydridfunktion und eine Carbonsäurefunktion umfasst, und/oder deren Tricarbonsäure- und/oder Ester- und/oder Säureanhydridchloridäquivalenten und mindestens des Diamin B der folgenden Formel II oder III: 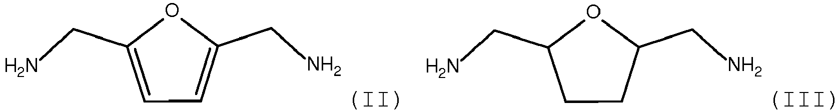 oder mindestens des Diisocyanats B' der folgenden Formel II' oder III': 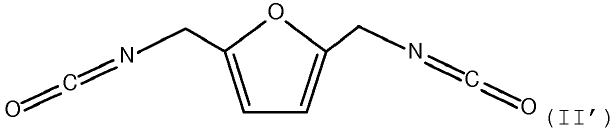 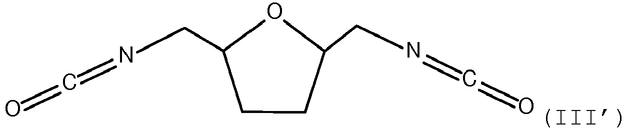 in Gegenwart mindestens eines Diamins C oder mindestens eines Diisocyanats C' der folgenden Formel IV oder IV':
NH₂-R₃-NH₂ (IV)
O=C=N-R₃-N=C=O (IV'),
wobei R₃ ein zweiwertiger aliphatischer, cycloaliphatischer oder arylaliphatischer, gesättigter und/oder ungesättigter, aromatischer oder alkylaromatischer Kohlenwasserstoffrest ist, der gegebenenfalls Heteroatome umfasst.

8. Polyamidsäure entsprechend der folgenden Formel V: 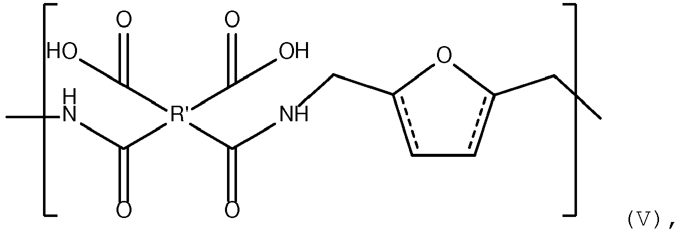 wobei R' wie in Anspruch 1 definiert ist.

9. Verfahren zur Herstellung des (Co)Polymers nach einem der Ansprüche 1 bis 7 durch Lösungspolymerisation im festem oder schmelzflüssigem Zustand mindestens einer Verbindung A und mindestens eines Diamins B, gegebenenfalls in Gegenwart mindestens eines Diamins C, wobei die Verbindungen A, B und C wie in einem der Ansprüche 2 bis 7 definiert sind, oder durch Lösungspolymerisation eines Ammoniumcarboxylatsalzes, das aus den Verbindungen A, B und C erhalten wird.

10. Verfahren zur Herstellung eines (Co)Polymers nach Anspruch 9, **dadurch gekennzeichnet, dass** es sich um eine Lösungspolymerisation handelt und dass es die folgenden Schritte umfasst:
- Umsetzen bei zwischen -20 und 120°C in einem polaren aprotischen Lösungsmittel oder einem phenolischen Lösungsmittel mindestens einer Verbindung A, die zwei Anhydridfunktionen umfasst, mit einem Diamin B und gegebenenfalls in Gegenwart mindestens eines Diamins C, wobei die Verbindungen A, B und C wie in einem der Ansprüche 2 bis 7 definiert sind, um ein Polyamidsäurezwischenprodukt der Formel V nach Anspruch 8 zu erhalten;
- Erhitzen auf eine Temperatur zwischen 120°C und 350°C und/oder Durchführung einer chemischen Dehydratisierung;
- Gewinnen des (Co)Polymers entweder durch Verdampfen des Lösungsmittels oder durch Ausfällung in einem Nicht-Lösungsmittel.

11. Ammoniumcarboxylatsalz, das aus einer oder mehreren Verbindungen A oder D und einem oder mehreren Diaminen B und gegebenenfalls einem oder mehreren Diaminen C erhalten wird, wobei die Verbindungen A, B, C und D wie in einem der Ansprüche 2 bis 7 definiert sind.

12. Zusammensetzung, die das (Co)Polymer nach einem der Ansprüche 1 bis 7 und Füllstoffe und/oder Additive umfasst.

13. Verfahren zur Herstellung eines Gegenstands auf der Basis des (Co)Polymers nach einem der Ansprüche 1 bis 7 mittels Formen durch Extrusion, Gießen oder Blasformen des (Co)Polymers in schmelzflüssiger Form, wenn es einen Schmelzpunkt unter 350°C aufweist, bei einem semikristallinen (Co)Polymer, oder eine Glasübergangstemperatur unter 300°C, bei einem amorphen (Co)Polymer.

14. Verfahren zur Herstellung eines Gegenstands auf der Basis des (Co)Polymers nach einem der Ansprüche 1 bis 7 durch Zyklisieren einer Polyamidsäure nach Anspruch 8 in fester Form oder in Form einer Lösung oder einer Dispersion in einem Lösungsmittel.

15. Gegenstand, der aus dem (Co)Polymer nach einem der Ansprüche 1 bis 7 oder der Zusammensetzung nach Anspruch 12 erhalten wird.

16. Gegenstand nach Anspruch 15, **dadurch gekennzeichnet, dass** es sich um ein Formteil, ein extrudiertes oder ein blasgeformtes Teil handelt.

17. Gegenstand nach Anspruch 16, **dadurch gekennzeichnet, dass** es sich um ein Spritzgussteil, einen Verbundstoff mit kontinuierlichen Fasern, eine Folie, eine Faser, einen Faden oder ein Filament, einen Schaum oder ein aus den Fasern, Fäden oder Filamenten gewebtes oder gestricktes Teil handelt.

## Claims

1. (Co)polymer comprising the recurring unit of following formula I: 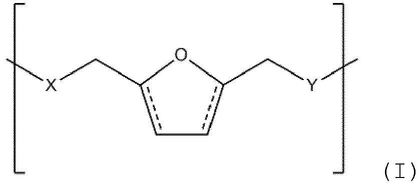 in which
- X is a divalent group of formula Ia or Ib below: 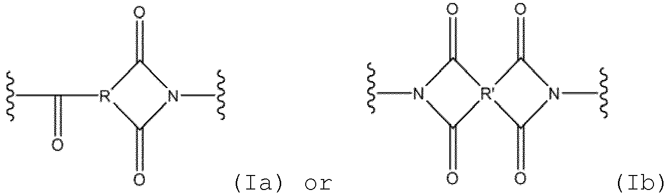 in which
• R is a trivalent hydrocarbon-based group selected from:
a saturated or unsaturated, aliphatic or cycloaliphatic group, optionally interrupted with one or more heteroatoms, and optionally substituted with one or more functional or non-functional groups;
an aromatic, alkylaromatic or arylaliphatic group, optionally interrupted with one or more heteroatoms, and optionally substituted with one or more functional or non-functional groups;
• R' is a tetravalent hydrocarbon-based group selected from:
a saturated or unsaturated, aliphatic or cycloaliphatic group, optionally interrupted with one or more heteroatoms, and optionally substituted with one or more functional or non-functional groups;
an aromatic, alkylaromatic or arylaliphatic group, optionally interrupted with one or more heteroatoms, and optionally substituted with one or more functional or non-functional groups;
- Y is:
• a covalent bond, when X corresponds to formula Ib,
• a divalent group of formula Ic or Id below, when X corresponds to formula Ia: 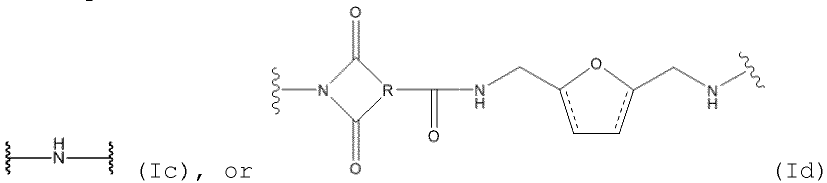 with R as defined above.

2. (Co)polymer according to Claim 1, obtained by polymerization of the following compounds:
- at least one compound A comprising two anhydride functions and/or its tetracarboxylic acid and/or ester equivalents, and
- at least one diamine B of formula II or III, 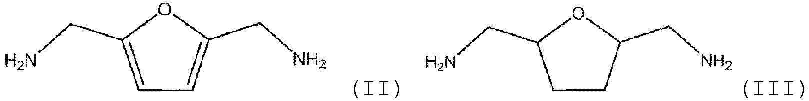 or
- at least one diisocyanate B' of formula II' or III' below: 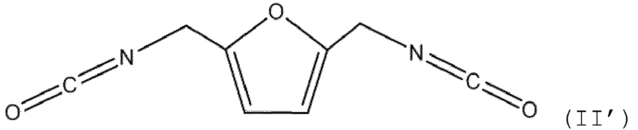 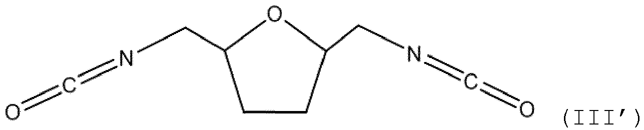 or
- at least one ammonium carboxylate salt obtained from the compounds A and B.

3. (Co)polymer according to Claim 2, **characterized in that** the compound A is pyromellitic anhydride or pyromellitic acid.

4. (Co)polymer according to Claim 1, obtained by polymerization of the following compounds:
- at least one compound D comprising an anhydride function and a carboxylic acid function and/or its tricarboxylic acid and/or ester and/or acid chloride anhydride equivalents; and
- at least one diamine B of formula II or III, 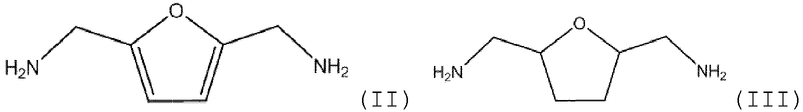 or
- at least one diisocyanate B' of formula II' or III' below: 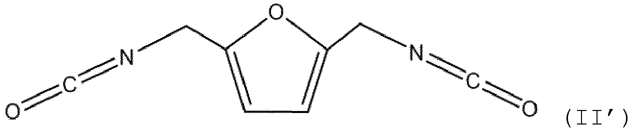 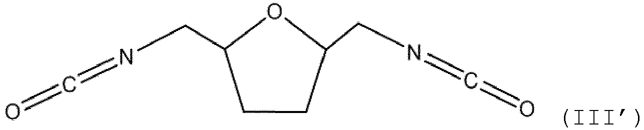 or
- at least one ammonium carboxylate salt obtained from the compounds D and B.

5. (Co)polymer according to Claim 4, **characterized in that** the compound D is selected from:
• trimellitic anhydride,
• tricarboxylic acids selected from trimellitic acid, tricarballylic acid, citric acid, aconitic acid, 1,2,4-butanetricarboxylic acid and 1,2,3-benzentricarboxylic acid,
• diacid monoesters, monoacid diesters or triesters of said tricarboxylic acids,
• trimellitic anhydride chloride.

6. (Co)polymer according to one of Claims 2 to 5, **characterized in that** the diamine B is a diamine of formula III below:

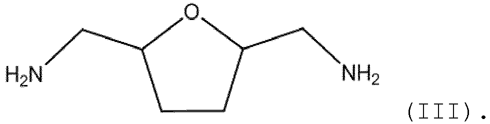

7. (Co)polymer according to any one of the preceding claims, **characterized in that** it is obtained by polymerization of at least a compound A comprising two anhydride functions and/or its tetracarboxylic acid and/or ester equivalents or D comprising an anhydride function and a carboxylic acid function and/or its tricarboxylic acid and/or ester and/or acid chloride anhydride equivalents and of at least the diamine B of formula II or III below: 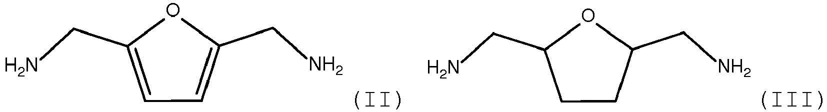 or of at least the diisocyanate B' of formula II' or III' below: 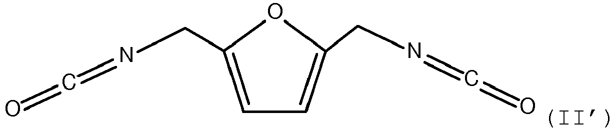 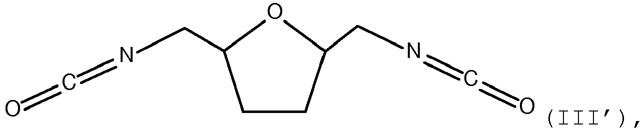 in the presence of at least one diamine C or of at least one diisocyanate C' of formula IV ou IV' below:
NH₂-R₃-NH₂ (IV)
O=C=N-R₃-N=C=O (IV')
with R₃ being a hydrocarbon-based divalent radical which is aliphatic, cycloaliphatic or arylaliphatic, and saturated and/or unsaturated, aromatic or alkylaromatic, and which optionally comprises heteroatoms.

8. Polyamic acid corresponding to formula V below: 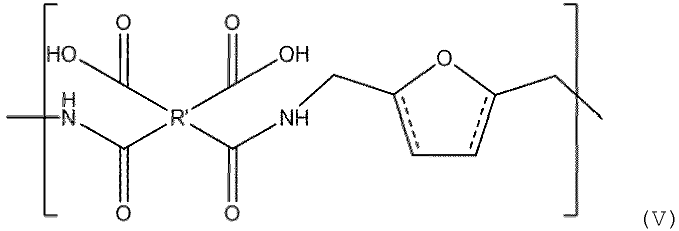 with R' being as defined in Claim 1.

9. Process for producing (co)polymer as defined in one of Claims 1 to 7 by solution polymerization, solid-state polymerization or melt polymerization of at least one compound A and of at least one diamine B, optionally in the presence of at least one diamine C, said compounds A, B and C being as defined in any one of Claims 2 to 7, or by solution polymerization of an ammonium carboxylate salt obtained from said compounds A, B and optionally C.

10. Process for producing (co)polymer according to claim 9, **characterized in that** it is a solution polymerization and that it comprises the following steps:
- at least one compound A comprising two anhydride functions is reacted, between -20 and 120°C, in a polar aprotic solvent or a phenolic solvent, with a diamine B, and optionally in the presence of at least one diamine C, said compounds A, B and C being as defined in one of claims 2 to 7, so as to form a polyamic acid intermediate of formula V as defined in claim 8;
- heating is carried out at a temperature of between 120°C and 350°C and/or a chemical dehydration is carried out;
- the (co)polymer is recovered either by evaporation of the solvent, or by precipitation from a non-solvent.

11. Ammonium carboxylate salt obtained from one or more compounds A or D and one or more diamines B and optionally one or more diamines C, said compounds A, B, C and D being as defined in any one of Claims 2 to 7.

12. Composition comprising the (co)polymer as defined in any one of claims 1 to 7 and fillers and/or additives.

13. Process for producing an article based on (co)polymer according to any one of Claims 1 to 7, by shaping by extrusion, molding or blow molding of said (co)polymer in molten form when it has a melting point below 350°C for a semi-crystalline (co)polymer or a glass transition temperature below 300°C for an amorphous (co)polymer.

14. Process for producing an article based on (co)polymer according to any one of Claims 1 to 7, by cyclization of a polyamic acid as defined in claim 8, in solid form or in the form of a solution or a dispersion in a solvent.

15. Article obtained from the (co)polymer as defined in any one of Claims 1 to 7 or from the composition as defined in Claim 12.

16. Article according to Claim 15, **characterized in that** it is a molded, extruded or blow-molded part.

17. Article according to Claim 16, **characterized in that** it is an injection-molded part, a composite comprising continuous fibers, a film, a fiber, a yarn or a filament, a foam or a part woven or knitted from said fibers, yarns or filaments.
